(19) Europäisches Patentamt / European Patent Office / Office européen des brevets

(11) **EP 2 565 276 A1**

(12) **EUROPEAN PATENT APPLICATION**

(43) Date of publication:
**06.03.2013 Bulletin 2013/10**

(51) Int Cl.:
***C12Q 1/42*** *(2006.01)*

(21) Application number: **11007082.8**

(22) Date of filing: **31.08.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung
der Wissenschaften e.V.
80539 München (DE)**

(72) Inventors:
• **Rauh, Daniel
67549 Worms (DE)**

• **Simard, Jeffrey Raymond
Salem
MA 01970 (US)**
• **Waldmann, Herbert
44265 Dortmund (DE)**

(74) Representative: **Vossius & Partner
Siebertstrasse 4
81675 München (DE)**

Remarks:
A request for correction of the description page 3 has been filed pursuant to Rule 139 EPC. A decision on the request will be taken during the proceedings before the Examining Division (Guidelines for Examination in the EPO, A-V, 3.).

(54) **Labeled phosphatases for the screening of inhibitors**

(57)    The present invention relates to a protein phosphatase containing exactly one label in (a) a structural element of said protein phosphatase, said structural element being selected from (a)(i) the C-terminal helix; and (a)(ii) the WPD loop; (b) wherein said label is a fluorophore, a spin label or an isotope label, (b)(i) wherein said fluorophore and said spin label are covalently bound to a thiol group or side chain amino group of an amino acid residue of said structural element, (b)(ii) wherein said isotope label is a moiety comprising one or more atoms which are isotopes with non-zero nuclear spin, said moiety being covalently bound to a thiol group or side chain amino group of an amino acid residue of said structural element, or said isotope label is an amino acid residue of said structural element, said amino acid residue comprising one or more atoms which are isotopes with non-zero nuclear spin, wherein said isotopes with non-zero nuclear spin are enriched as compared to the natural occurrence thereof.

EP 2 565 276 A1

SEQUENCE LISTING

<110> Max-Planck-Gesellschaft zur Förderung der Wissenschaften e.V.

<120> LABELED PHOSPHATASES FOR THE SCREENING OF INHIBITORS

<130> T1081 EP

<160> 8

<170> PatentIn version 3.4

<210> 1
<211> 435
<212> PRT
<213> homo sapiens

<400> 1

```
Met Glu Met Glu Lys Glu Phe Glu Gln Ile Asp Lys Ser Gly Ser Trp
1               5                   10                  15

Ala Ala Ile Tyr Gln Asp Ile Arg His Glu Ala Ser Asp Phe Pro Cys
            20                  25                  30

Arg Val Ala Lys Leu Pro Lys Asn Lys Asn Arg Asn Arg Tyr Arg Asp
            35                  40                  45

Val Ser Pro Phe Asp His Ser Arg Ile Lys Leu His Gln Glu Asp Asn
        50                  55                  60

Asp Tyr Ile Asn Ala Ser Leu Ile Lys Met Glu Glu Ala Gln Arg Ser
65                  70                  75                  80

Tyr Ile Leu Thr Gln Gly Pro Leu Pro Asn Thr Cys Gly His Phe Trp
                85                  90                  95

Glu Met Val Trp Glu Gln Lys Ser Arg Gly Val Val Met Leu Asn Arg
            100                 105                 110

Val Met Glu Lys Gly Ser Leu Lys Cys Ala Gln Tyr Trp Pro Gln Lys
            115                 120                 125

Glu Glu Lys Glu Met Ile Phe Glu Asp Thr Asn Leu Lys Leu Thr Leu
        130                 135                 140

Ile Ser Glu Asp Ile Lys Ser Tyr Tyr Thr Val Arg Gln Leu Glu Leu
145                 150                 155                 160

Glu Asn Leu Thr Thr Gln Glu Thr Arg Glu Ile Leu His Phe His Tyr
                165                 170                 175

Thr Thr Trp Pro Asp Phe Gly Val Pro Glu Ser Pro Ala Ser Phe Leu
                180                 185                 190
```

```
Asn Phe Leu Phe Lys Val Arg Glu Ser Gly Ser Leu Ser Pro Glu His
        195                 200             205

Gly Pro Val Val Val His Cys Ser Ala Gly Ile Gly Arg Ser Gly Thr
    210                 215                 220

Phe Cys Leu Ala Asp Thr Cys Leu Leu Leu Met Asp Lys Arg Lys Asp
225                 230                 235                 240

Pro Ser Ser Val Asp Ile Lys Lys Val Leu Leu Glu Met Arg Lys Phe
                245                 250                 255

Arg Met Gly Leu Ile Gln Thr Ala Asp Gln Leu Arg Phe Ser Tyr Leu
            260                 265                 270

Ala Val Ile Glu Gly Ala Lys Phe Ile Met Gly Asp Ser Ser Val Gln
            275                 280                 285

Asp Gln Trp Lys Glu Leu Ser His Glu Asp Leu Glu Pro Pro Pro Glu
    290                 295                 300

His Ile Pro Pro Pro Pro Arg Pro Pro Lys Arg Ile Leu Glu Pro His
305                 310                 315                 320

Asn Gly Lys Cys Arg Glu Phe Phe Pro Asn His Gln Trp Val Lys Glu
                325                 330                 335

Glu Thr Gln Glu Asp Lys Asp Cys Pro Ile Lys Glu Glu Lys Gly Ser
            340                 345                 350

Pro Leu Asn Ala Ala Pro Tyr Gly Ile Glu Ser Met Ser Gln Asp Thr
        355                 360                 365

Glu Val Arg Ser Arg Val Val Gly Gly Ser Leu Arg Gly Ala Gln Ala
    370                 375                 380

Ala Ser Pro Ala Lys Gly Glu Pro Ser Leu Pro Glu Lys Asp Glu Asp
385                 390                 395                 400

His Ala Leu Ser Tyr Trp Lys Pro Phe Leu Val Asn Met Cys Val Ala
                405                 410                 415

Thr Val Leu Thr Ala Gly Ala Tyr Leu Cys Tyr Arg Phe Leu Phe Asn
            420                 425                 430

Ser Asn Thr
        435


<210>   2
<211>   415
<212>   PRT
<213>   homo sapiens
```

<400> 2

Met Pro Thr Thr Ile Glu Arg Glu Phe Glu Glu Leu Asp Thr Gln Arg
1               5               10                  15

Arg Trp Gln Pro Leu Tyr Leu Glu Ile Arg Asn Glu Ser His Asp Tyr
            20              25                  30

Pro His Arg Val Ala Lys Phe Pro Glu Asn Arg Asn Arg Asn Arg Tyr
        35              40                  45

Arg Asp Val Ser Pro Tyr Asp His Ser Arg Val Lys Leu Gln Asn Ala
        50              55                  60

Glu Asn Asp Tyr Ile Asn Ala Ser Leu Val Asp Ile Glu Glu Ala Gln
65              70              75                  80

Arg Ser Tyr Ile Leu Thr Gln Gly Pro Leu Pro Asn Thr Cys Cys His
            85              90                  95

Phe Trp Leu Met Val Trp Gln Gln Lys Thr Lys Ala Val Val Met Leu
            100             105                 110

Asn Arg Ile Val Glu Lys Glu Ser Val Lys Cys Ala Gln Tyr Trp Pro
        115             120                 125

Thr Asp Asp Gln Glu Met Leu Phe Lys Glu Thr Gly Phe Ser Val Lys
    130             135                 140

Leu Leu Ser Glu Asp Val Lys Ser Tyr Tyr Thr Val His Leu Leu Gln
145             150             155                 160

Leu Glu Asn Ile Asn Ser Gly Glu Thr Arg Thr Ile Ser His Phe His
            165             170                 175

Tyr Thr Thr Trp Pro Asp Phe Gly Val Pro Glu Ser Pro Ala Ser Phe
            180             185                 190

Leu Asn Phe Leu Phe Lys Val Arg Glu Ser Gly Ser Leu Asn Pro Asp
        195             200                 205

His Gly Pro Ala Val Ile His Cys Ser Ala Gly Ile Gly Arg Ser Gly
    210             215                 220

Thr Phe Ser Leu Val Asp Thr Cys Leu Val Leu Met Glu Lys Gly Asp
225             230             235                 240

Asp Ile Asn Ile Lys Gln Val Leu Leu Asn Met Arg Lys Tyr Arg Met
            245             250                 255

Gly Leu Ile Gln Thr Pro Asp Gln Leu Arg Phe Ser Tyr Met Ala Ile

EP 2 565 276 A1

```
                260                     265                     270

        Ile Glu Gly Ala Lys Cys Ile Lys Gly Asp Ser Ser Ile Gln Lys Arg
                275             280             285

        Trp Lys Glu Leu Ser Lys Glu Asp Leu Ser Pro Ala Phe Asp His Ser
            290             295             300

        Pro Asn Lys Ile Met Thr Glu Lys Tyr Asn Gly Asn Arg Ile Gly Leu
        305             310             315             320

        Glu Glu Glu Lys Leu Thr Gly Asp Arg Cys Thr Gly Leu Ser Ser Lys
                325             330             335

        Met Gln Asp Thr Met Glu Glu Asn Ser Glu Ser Ala Leu Arg Lys Arg
                340             345             350

        Ile Arg Glu Asp Arg Lys Ala Thr Thr Ala Gln Lys Val Gln Gln Met
                355             360             365

        Lys Gln Arg Leu Asn Glu Asn Glu Arg Lys Arg Lys Arg Trp Leu Tyr
            370             375             380

        Trp Gln Pro Ile Leu Thr Lys Met Gly Phe Met Ser Val Ile Leu Val
        385             390             395             400

        Gly Ala Phe Val Gly Trp Thr Leu Phe Phe Gln Gln Asn Ala Leu
                405             410             415
```

```
<210>   3
<211>   276
<212>   PRT
<213>   Mycobacterium tuberculosis

<400>   3
```

```
        Met Ala Val Arg Glu Leu Pro Gly Ala Trp Asn Phe Arg Asp Val Ala
        1               5               10              15

        Asp Thr Ala Thr Ala Leu Arg Pro Gly Arg Leu Phe Arg Ser Ser Glu
                20              25              30

        Leu Ser Arg Leu Asp Asp Ala Gly Arg Ala Thr Leu Arg Arg Leu Gly
                35              40              45

        Ile Thr Asp Val Ala Asp Leu Arg Ser Ser Arg Glu Val Ala Arg Arg
            50              55              60

        Gly Pro Gly Arg Val Pro Asp Gly Ile Asp Val His Leu Leu Pro Phe
        65              70              75              80

        Pro Asp Leu Ala Asp Asp Asp Ala Asp Asp Ser Ala Pro His Glu Thr
                85              90              95
```

```
Ala Phe Lys Arg Leu Leu Thr Asn Asp Gly Ser Asn Gly Glu Ser Gly
            100                 105             110

Glu Ser Ser Gln Ser Ile Asn Asp Ala Ala Thr Arg Tyr Met Thr Asp
        115                 120             125

Glu Tyr Arg Gln Phe Pro Thr Arg Asn Gly Ala Gln Arg Ala Leu His
    130                 135             140

Arg Val Val Thr Leu Leu Ala Ala Gly Arg Pro Val Leu Thr His Cys
145                 150                 155             160

Phe Ala Gly Lys Asp Arg Thr Gly Phe Val Val Ala Leu Val Leu Glu
                165                 170             175

Ala Val Gly Leu Asp Arg Asp Val Ile Val Ala Asp Tyr Leu Arg Ser
            180                 185             190

Asn Asp Ser Val Pro Gln Leu Arg Ala Arg Ile Ser Glu Met Ile Gln
        195                 200             205

Gln Arg Phe Asp Thr Glu Leu Ala Pro Glu Val Val Thr Phe Thr Lys
    210                 215             220

Ala Arg Leu Ser Asp Gly Val Leu Gly Val Arg Ala Glu Tyr Leu Ala
225                 230                 235             240

Ala Ala Arg Gln Thr Ile Asp Glu Thr Tyr Gly Ser Leu Gly Gly Tyr
                245                 250             255

Leu Arg Asp Ala Gly Ile Ser Gln Ala Thr Val Asn Arg Met Arg Gly
            260                 265             270

Val Leu Leu Gly
            275
```

```
<210>  4
<211>  595
<212>  PRT
<213>  homo sapiens

<400>  4
```

```
Met Val Arg Trp Phe His Arg Asp Leu Ser Gly Leu Asp Ala Glu Thr
1               5                 10              15

Leu Leu Lys Gly Arg Gly Val His Gly Ser Phe Leu Ala Arg Pro Ser
            20                  25              30

Arg Lys Asn Gln Gly Asp Phe Ser Leu Ser Val Arg Val Gly Asp Gln
        35                  40              45
```

EP 2 565 276 A1

Val Thr His Ile Arg Ile Gln Asn Ser Gly Asp Phe Tyr Asp Leu Tyr
    50                      55                  60

Gly Gly Glu Lys Phe Ala Thr Leu Thr Glu Leu Val Glu Tyr Tyr Thr
65                  70                  75                      80

Gln Gln Gln Gly Val Leu Gln Asp Arg Asp Gly Thr Ile Ile His Leu
                85                  90                  95

Lys Tyr Pro Leu Asn Cys Ser Asp Pro Thr Ser Glu Arg Trp Tyr His
            100                 105                 110

Gly His Met Ser Gly Gly Gln Ala Glu Thr Leu Leu Gln Ala Lys Gly
        115                 120                 125

Glu Pro Trp Thr Phe Leu Val Arg Glu Ser Leu Ser Gln Pro Gly Asp
    130                 135                 140

Phe Val Leu Ser Val Leu Ser Asp Gln Pro Lys Ala Gly Pro Gly Ser
145                 150                 155                     160

Pro Leu Arg Val Thr His Ile Lys Val Met Cys Glu Gly Gly Arg Tyr
            165                 170                 175

Thr Val Gly Gly Leu Glu Thr Phe Asp Ser Leu Thr Asp Leu Val Glu
            180                 185                 190

His Phe Lys Lys Thr Gly Ile Glu Glu Ala Ser Gly Ala Phe Val Tyr
        195                 200                 205

Leu Arg Gln Pro Tyr Tyr Ala Thr Arg Val Asn Ala Ala Asp Ile Glu
    210                 215                 220

Asn Arg Val Leu Glu Leu Asn Lys Lys Gln Glu Ser Glu Asp Thr Ala
225                 230                 235                     240

Lys Ala Gly Phe Trp Glu Glu Phe Glu Ser Leu Gln Lys Gln Glu Val
            245                 250                 255

Lys Asn Leu His Gln Arg Leu Glu Gly Gln Arg Pro Glu Asn Lys Gly
        260                 265                 270

Lys Asn Arg Tyr Lys Asn Ile Leu Pro Phe Asp His Ser Arg Val Ile
        275                 280                 285

Leu Gln Gly Arg Asp Ser Asn Ile Pro Gly Ser Asp Tyr Ile Asn Ala
    290                 295                 300

Asn Tyr Ile Lys Asn Gln Leu Leu Gly Pro Asp Glu Asn Ala Lys Thr
305                 310                 315                     320

20

Tyr Ile Ala Ser Gln Gly Cys Leu Glu Ala Thr Val Asn Asp Phe Trp
                325             330             335

Gln Met Ala Trp Gln Glu Asn Ser Arg Val Ile Val Met Thr Thr Arg
            340             345             350

Glu Val Glu Lys Gly Arg Asn Lys Cys Val Pro Tyr Trp Pro Glu Val
        355             360             365

Gly Met Gln Arg Ala Tyr Gly Pro Tyr Ser Val Thr Asn Cys Gly Glu
    370             375             380

His Asp Thr Thr Glu Tyr Lys Leu Arg Thr Leu Gln Val Ser Pro Leu
385             390             395             400

Asp Asn Gly Asp Leu Ile Arg Glu Ile Trp His Tyr Gln Tyr Leu Ser
            405             410             415

Trp Pro Asp His Gly Val Pro Ser Glu Pro Gly Gly Val Leu Ser Phe
            420             425             430

Leu Asp Gln Ile Asn Gln Arg Gln Glu Ser Leu Pro His Ala Gly Pro
            435             440             445

Ile Ile Val His Cys Ser Ala Gly Ile Gly Arg Thr Gly Thr Ile Ile
    450             455             460

Val Ile Asp Met Leu Met Glu Asn Ile Ser Thr Lys Gly Leu Asp Cys
465             470             475             480

Asp Ile Asp Ile Gln Lys Thr Ile Gln Met Val Arg Ala Gln Arg Ser
            485             490             495

Gly Met Val Gln Thr Glu Ala Gln Tyr Lys Phe Ile Tyr Val Ala Ile
        500             505             510

Ala Gln Phe Ile Glu Thr Thr Lys Lys Lys Leu Glu Val Leu Gln Ser
        515             520             525

Gln Lys Gly Gln Glu Ser Glu Tyr Gly Asn Ile Thr Tyr Pro Pro Ala
    530             535             540

Met Lys Asn Ala His Ala Lys Ala Ser Arg Thr Ser Ser Lys His Lys
545             550             555             560

Glu Asp Val Tyr Glu Asn Leu His Thr Lys Asn Lys Arg Glu Glu Lys
            565             570             575

Val Lys Lys Gln Arg Ser Ala Asp Lys Glu Lys Ser Lys Gly Ser Leu
    580             585             590

Lys Arg Lys
595

<210> 5
<211> 597
<212> PRT
<213> homo sapiens

<400> 5

Met Thr Ser Arg Arg Trp Phe His Pro Asn Ile Thr Gly Val Glu Ala
1               5                   10                  15

Glu Asn Leu Leu Leu Thr Arg Gly Val Asp Gly Ser Phe Leu Ala Arg
            20                  25                  30

Pro Ser Lys Ser Asn Pro Gly Asp Phe Thr Leu Ser Val Arg Arg Asn
            35                  40                  45

Gly Ala Val Thr His Ile Lys Ile Gln Asn Thr Gly Asp Tyr Tyr Asp
        50                  55                  60

Leu Tyr Gly Gly Glu Lys Phe Ala Thr Leu Ala Glu Leu Val Gln Tyr
65                  70                  75                  80

Tyr Met Glu His His Gly Gln Leu Lys Glu Lys Asn Gly Asp Val Ile
                85                  90                  95

Glu Leu Lys Tyr Pro Leu Asn Cys Ala Asp Pro Thr Ser Glu Arg Trp
            100                 105                 110

Phe His Gly His Leu Ser Gly Lys Glu Ala Glu Lys Leu Leu Thr Glu
            115                 120                 125

Lys Gly Lys His Gly Ser Phe Leu Val Arg Glu Ser Gln Ser His Pro
        130                 135                 140

Gly Asp Phe Val Leu Ser Val Arg Thr Gly Asp Asp Lys Gly Glu Ser
145                 150                 155                 160

Asn Asp Gly Lys Ser Lys Val Thr His Val Met Ile Arg Cys Gln Glu
                165                 170                 175

Leu Lys Tyr Asp Val Gly Gly Gly Glu Arg Phe Asp Ser Leu Thr Asp
            180                 185                 190

Leu Val Glu His Tyr Lys Lys Asn Pro Met Val Glu Thr Leu Gly Thr
            195                 200                 205

Val Leu Gln Leu Lys Gln Pro Leu Asn Thr Thr Arg Ile Asn Ala Ala
            210                 215                 220

```
Glu Ile Glu Ser Arg Val Arg Glu Leu Ser Lys Leu Ala Glu Thr Thr
225                 230             235                 240

Asp Lys Val Lys Gln Gly Phe Trp Glu Glu Phe Glu Thr Leu Gln Gln
                245             250                 255

Gln Glu Cys Lys Leu Leu Tyr Ser Arg Lys Glu Gly Gln Arg Gln Glu
                260             265             270

Asn Lys Asn Lys Asn Arg Tyr Lys Asn Ile Leu Pro Phe Asp His Thr
                275             280             285

Arg Val Val Leu His Asp Gly Asp Pro Asn Glu Pro Val Ser Asp Tyr
    290             295                 300

Ile Asn Ala Asn Ile Ile Met Pro Glu Phe Glu Thr Lys Cys Asn Asn
305             310             315                 320

Ser Lys Pro Lys Lys Ser Tyr Ile Ala Thr Gln Gly Cys Leu Gln Asn
                325             330             335

Thr Val Asn Asp Phe Trp Arg Met Val Phe Gln Glu Asn Ser Arg Val
                340             345             350

Ile Val Met Thr Thr Lys Glu Val Glu Arg Gly Lys Ser Lys Cys Val
            355             360             365

Lys Tyr Trp Pro Asp Glu Tyr Ala Leu Lys Glu Tyr Gly Val Met Arg
    370             375             380

Val Arg Asn Val Lys Glu Ser Ala Ala His Asp Tyr Thr Leu Arg Glu
385             390             395                 400

Leu Lys Leu Ser Lys Val Gly Gln Ala Leu Leu Gln Gly Asn Thr Glu
                405             410             415

Arg Thr Val Trp Gln Tyr His Phe Arg Thr Trp Pro Asp His Gly Val
            420             425             430

Pro Ser Asp Pro Gly Gly Val Leu Asp Phe Leu Glu Glu Val His His
            435             440             445

Lys Gln Glu Ser Ile Met Asp Ala Gly Pro Val Val Val His Cys Ser
    450             455             460

Ala Gly Ile Gly Arg Thr Gly Thr Phe Ile Val Ile Asp Ile Leu Ile
465             470             475             480

Asp Ile Ile Arg Glu Lys Gly Val Asp Cys Asp Ile Asp Val Pro Lys
            485             490             495
```

23

EP 2 565 276 A1

Thr Ile Gln Met Val Arg Ser Gln Arg Ser Gly Met Val Gln Thr Glu
500 505 510

Ala Gln Tyr Arg Phe Ile Tyr Met Ala Val Gln His Tyr Ile Glu Thr
515 520 525

Leu Gln Arg Arg Ile Glu Glu Glu Gln Lys Ser Lys Arg Lys Gly His
530 535 540

Glu Tyr Thr Asn Ile Lys Tyr Ser Leu Ala Asp Gln Thr Ser Gly Asp
545 550 555 560

Gln Ser Pro Leu Pro Pro Cys Thr Pro Thr Pro Pro Cys Ala Glu Met
565 570 575

Arg Glu Asp Ser Ala Arg Val Tyr Glu Asn Val Gly Leu Met Gln Gln
580 585 590

Gln Lys Ser Phe Arg
595

<210> 6
<211> 321
<212> PRT
<213> artificial sequence

<220>
<223> /note="Description of artificial sequence:PTP1B WPD-Loop Flip"

<400> 6

Met Glu Met Glu Lys Glu Phe Glu Gln Ile Asp Lys Ser Gly Ser Trp
1 5 10 15

Ala Ala Ile Tyr Gln Asp Ile Arg His Glu Ala Ser Asp Phe Pro Ser
20 25 30

Arg Val Ala Lys Leu Pro Lys Asn Lys Asn Arg Asn Arg Tyr Arg Asp
35 40 45

Val Ser Pro Phe Asp His Ser Arg Ile Lys Leu His Gln Glu Asp Asn
50 55 60

Asp Tyr Ile Asn Ala Ser Leu Ile Lys Met Glu Glu Ala Gln Arg Ser
65 70 75 80

Tyr Ile Leu Thr Gln Gly Pro Leu Pro Asn Thr Ser Gly His Phe Trp
85 90 95

Glu Met Val Trp Glu Gln Lys Ser Arg Gly Val Val Met Leu Asn Arg
100 105 110

Val Met Glu Lys Gly Ser Leu Lys Ser Ala Gln Tyr Trp Pro Gln Lys
115 120 125

24

Glu Glu Lys Glu Met Ile Phe Glu Asp Thr Asn Leu Lys Leu Thr Leu
    130             135             140

Ile Ser Glu Asp Ile Lys Ser Tyr Tyr Thr Val Arg Gln Leu Glu Leu
145             150             155             160

Glu Asn Leu Thr Thr Gln Glu Thr Arg Glu Ile Leu His Phe His Tyr
            165             170             175

Thr Thr Trp Pro Asp Cys Gly Val Pro Glu Ser Pro Ala Ser Phe Leu
            180             185             190

Asn Phe Leu Phe Lys Val Arg Glu Ser Gly Ser Leu Ser Pro Glu His
        195             200             205

Gly Pro Val Val Val His Ser Ser Ala Gly Ile Gly Arg Ser Gly Thr
    210             215             220

Phe Cys Leu Ala Asp Thr Cys Leu Leu Leu Met Asp Lys Arg Lys Asp
225             230             235             240

Pro Ser Ser Val Asp Ile Lys Lys Val Leu Leu Glu Met Arg Lys Phe
            245             250             255

Arg Met Gly Leu Ile Gln Thr Ala Asp Gln Leu Arg Phe Ser Tyr Leu
        260             265             270

Ala Val Ile Glu Gly Ala Lys Phe Ile Met Gly Asp Ser Ser Val Gln
    275             280             285

Asp Gln Trp Lys Glu Leu Ser His Glu Asp Leu Glu Pro Pro Pro Glu
    290             295             300

His Ile Pro Pro Pro Pro Arg Pro Pro Lys Arg Ile Leu Glu Pro His
305             310             315             320

Asn

<210>  7
<211>  321
<212>  PRT
<213>  artificial sequence

<220>
<223>  /note="Description of artificial sequence:PTP1B helix alpha7
       Flip"

<400>  7

Met Glu Met Glu Lys Glu Phe Glu Gln Ile Asp Lys Ser Gly Ser Trp
1               5               10              15

```
Ala Ala Ile Tyr Gln Asp Ile Arg His Glu Ala Ser Asp Phe Pro Ser
            20              25              30

Arg Val Ala Lys Leu Pro Lys Asn Lys Asn Arg Asn Arg Tyr Arg Asp
            35              40              45

Val Ser Pro Phe Asp His Ser Arg Ile Lys Leu His Gln Glu Asp Asn
    50              55              60

Asp Tyr Ile Asn Ala Ser Leu Ile Lys Met Glu Glu Ala Gln Arg Ser
65              70              75              80

Tyr Ile Leu Thr Gln Gly Pro Leu Pro Asn Thr Ser Gly His Phe Trp
            85              90              95

Glu Met Val Trp Glu Gln Lys Ser Arg Gly Val Val Met Leu Asn Arg
            100             105             110

Val Met Glu Lys Gly Ser Leu Lys Ser Ala Gln Tyr Trp Pro Gln Lys
        115             120             125

Glu Glu Lys Glu Met Ile Phe Glu Asp Thr Asn Leu Lys Leu Thr Leu
    130             135                 140

Ile Ser Glu Asp Ile Lys Ser Tyr Tyr Thr Val Arg Gln Leu Glu Leu
145             150             155             160

Glu Asn Leu Thr Thr Gln Glu Thr Arg Glu Ile Leu His Phe His Tyr
            165             170             175

Thr Thr Trp Pro Asp Phe Gly Val Pro Glu Ser Pro Ala Ser Phe Leu
            180             185             190

Asn Phe Leu Phe Lys Val Arg Glu Ser Gly Ser Leu Ser Pro Glu His
        195             200             205

Gly Pro Val Val Val His Ser Ser Ala Gly Ile Gly Arg Ser Gly Thr
    210             215             220

Phe Cys Leu Ala Asp Thr Cys Leu Leu Leu Met Asp Lys Arg Lys Asp
225             230             235             240

Pro Ser Ser Val Asp Ile Lys Lys Val Leu Leu Glu Met Arg Lys Phe
            245             250             255

Arg Met Gly Leu Ile Gln Thr Ala Asp Gln Leu Arg Phe Ser Tyr Leu
        260             265             270

Ala Val Ile Glu Gly Ala Lys Phe Ile Met Gly Asp Ser Ser Val Gln
        275             280             285
```

Asp Gln Trp Lys Glu Cys Ser His Glu Asp Leu Glu Pro Pro Pro Glu
    290                 295             300

His Ile Pro Pro Pro Pro Arg Pro Pro Lys Arg Ile Leu Glu Pro His
305             310                 315             320

Asn

<210> 8
<211> 321
<212> PRT
<213> artificial sequence

<220>
<223> /note="Description of artificial sequence:truncated PTP1B wild
      type"

<400> 8

Met Glu Met Glu Lys Glu Phe Glu Gln Ile Asp Lys Ser Gly Ser Trp
1               5                   10              15

Ala Ala Ile Tyr Gln Asp Ile Arg His Glu Ala Ser Asp Phe Pro Cys
            20              25              30

Arg Val Ala Lys Leu Pro Lys Asn Lys Asn Arg Asn Arg Tyr Arg Asp
        35              40              45

Val Ser Pro Phe Asp His Ser Arg Ile Lys Leu His Gln Glu Asp Asn
    50              55              60

Asp Tyr Ile Asn Ala Ser Leu Ile Lys Met Glu Glu Ala Gln Arg Ser
65              70              75              80

Tyr Ile Leu Thr Gln Gly Pro Leu Pro Asn Thr Cys Gly His Phe Trp
            85              90              95

Glu Met Val Trp Glu Gln Lys Ser Arg Gly Val Val Met Leu Asn Arg
            100             105             110

Val Met Glu Lys Gly Ser Leu Lys Cys Ala Gln Tyr Trp Pro Gln Lys
        115             120             125

Glu Glu Lys Glu Met Ile Phe Glu Asp Thr Asn Leu Lys Leu Thr Leu
    130             135             140

Ile Ser Glu Asp Ile Lys Ser Tyr Tyr Thr Val Arg Gln Leu Glu Leu
145             150             155             160

Glu Asn Leu Thr Thr Gln Glu Thr Arg Glu Ile Leu His Phe His Tyr
            165             170             175

27

```
Thr Thr Trp Pro Asp Phe Gly Val Pro Glu Ser Pro Ala Ser Phe Leu
            180                 185             190

Asn Phe Leu Phe Lys Val Arg Glu Ser Gly Ser Leu Ser Pro Glu His
        195                 200             205

Gly Pro Val Val Val His Cys Ser Ala Gly Ile Gly Arg Ser Gly Thr
    210                 215             220

Phe Cys Leu Ala Asp Thr Cys Leu Leu Leu Met Asp Lys Arg Lys Asp
225                 230             235                 240

Pro Ser Ser Val Asp Ile Lys Lys Val Leu Leu Glu Met Arg Lys Phe
            245                 250                 255

Arg Met Gly Leu Ile Gln Thr Ala Asp Gln Leu Arg Phe Ser Tyr Leu
            260                 265             270

Ala Val Ile Glu Gly Ala Lys Phe Ile Met Gly Asp Ser Ser Val Gln
            275                 280             285

Asp Gln Trp Lys Glu Leu Ser His Glu Asp Leu Glu Pro Pro Pro Glu
    290                 295             300

His Ile Pro Pro Pro Pro Arg Pro Pro Lys Arg Ile Leu Glu Pro His
305                 310             315                 320

Asn
```

**Description**

The present invention relates to a protein phosphatase containing exactly one label in (a) a structural element of said protein phosphatase, said structural element being selected from (a)(i) the C-terminal helix; and (a)(ii) the WPD loop; (b) wherein said label is a fluorophore, a spin label or an isotope label, (b)(i) wherein said fluorophore and said spin label are covalently bound to a thiol group or side chain amino group of an amino acid residue of said structural element, (b)(ii) wherein said isotope label is a moiety comprising one or more atoms which are isotopes with non-zero nuclear spin, said moiety being covalently bound to a thiol group or side chain amino group of an amino acid residue of said structural element, or said isotope label is an amino acid residue of said structural element, said amino acid residue comprising one or more atoms which are isotopes with non-zero nuclear spin, wherein said isotopes with non-zero nuclear spin are enriched as compared to the natural occurrence thereof.

In this specification, a number of documents including patent applications and manufacturer's manuals are cited. The disclosure of these documents, while not considered relevant for the patentability of this invention, is herewith incorporated by reference in its entirety. More specifically, all reference documents are incorporated by reference to the same extent as if each individual document was specifically and individually indicated to be incorporated by reference.

Protein phosphatases are considered valuable drug targets. Although phosphatases and their inhibitors are heavily investigated, the development of clinically relevant phosphatase inhibitors faces major road blocks and is often plagued by limited selectivity and unfavorable pharmacokinetics. Until now no phosphatase inhibitor was approved by the FDA for the treatment of human diseases and calls for the development of next generation drugs and therapeutics which circumvent these major roadblocks in phosphatase inhibitor research. In case of PTP1B, a highly sought drug target, the reason for these limitations is frequent unwanted cross-reactivity with T-Cell PTP (77% identical to PTP1 b). Interestingly, PTP1b b and TCPTP both have an allosteric pocket but only PTP1b has a critical Phe residue at this site which is required for high affinity allosteric inhibitor binding. The corresponding position in TCPTP is a Cys. Methods which can identify specific allosteric PTP1b inhibitors may overcome current problems encountered in the pharmaceutical industry with inhibiting PTP1 b.

Therefore, the technical problem underlying the present invention may be seen in the provision of alternative or improved means and methods for identifying compounds binding to and, in particular, inhibiting protein phosphatases, preferably in a specific manner.

Accordingly, the present invention provides a protein phosphatase containing exactly one label in (a) a structural element of said protein phosphatase, said structural element being selected from (a)(i) the C-terminal helix; and (a)(ii) the WPD loop; (b) wherein said label is a fluorophore, a spin label or an isotope label, (b)(i) wherein said fluorophore and said spin label are covalently bound to a thiol group or side chain amino group of an amino acid residue of said structural element, (b)(ii) wherein said isotope label is a moiety comprising one or more atoms which are isotopes with non-zero nuclear spin, said moiety being covalently bound to a thiol group or side chain amino group of an amino acid residue of said structural element, or said isotope label is an amino acid residue of said structural element, said amino acid residue comprising one or more atoms which are isotopes with non-zero nuclear spin, wherein said isotopes with non-zero nuclear spin are enriched as compared to the natural occurrence thereof.

The term "phosphatase" is well-known in the art and refers to a type of enzyme that removes phosphate groups from a substrate, such as proteins. Phosphatases are classified under the enzyme commission (EC) number 3.1.3. Phosphatases which dephosphorylate proteins are also referred to as protein phosphatases. Protein phosphatases can be viewed as counterparts of protein kinases. In many instances, the activity of a given target protein is governed by its phosphorylation status. The phosphorylation status in turn is controlled by the activities of a cognate protein kinase on the one hand and of a cognate protein phosphatase on the other hand.

According to substrate specificity, protein phosphatases can be subdivided into tyrosine-specific phosphatases (e.g. PTP1B), serine/threonine-specific phosphatases (e.g. PP2C (PPP2CA)), dual-specificity phosphatases (e.g. DUSP1), Histidine Phosphatase (e.g. PHP) and lipid phosphatases (to the extent they recognize protein substrates; e.g. PTEN).

Protein phosphatases may also be classified according to presence or absence of certain secondary structure elements within the tertiary structure of a given protein phosphatase. For example, protein tyrosine phosphatases (herein also referred to as tyrosine-specific phosphatases) typically contain a loop which is well-known in the art as "WPD loop". While the presence of the WPD loop is a feature characterizing tyrosine-specific phosphatases, its presence is not necessarily confined to this subclass of protein phosphatases.

Certain protein phosphatases are characterized by a C-terminal helix which is commonly referred to in the art as α7 helix.

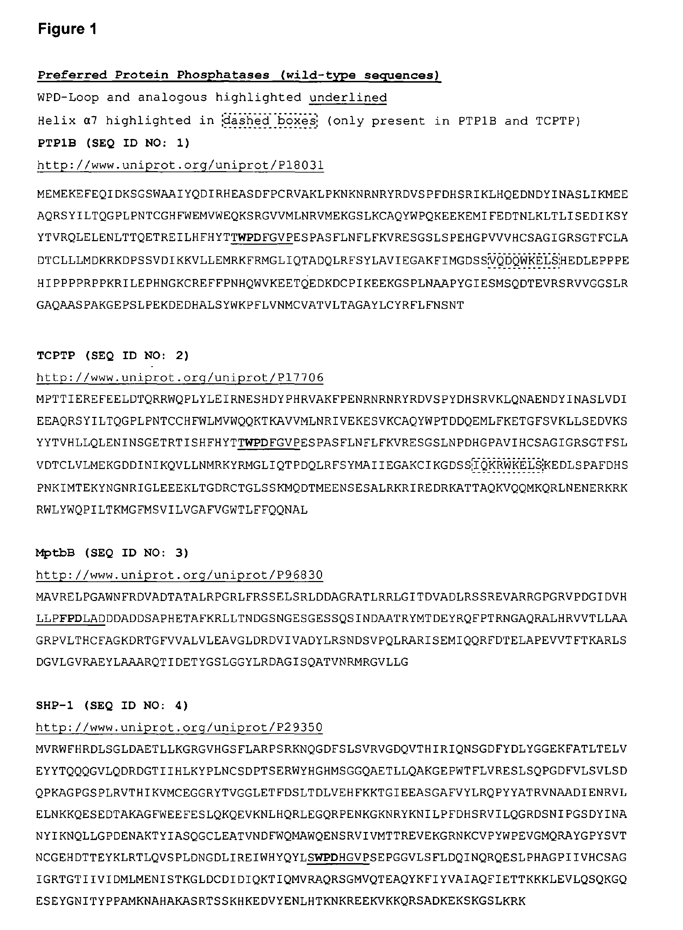

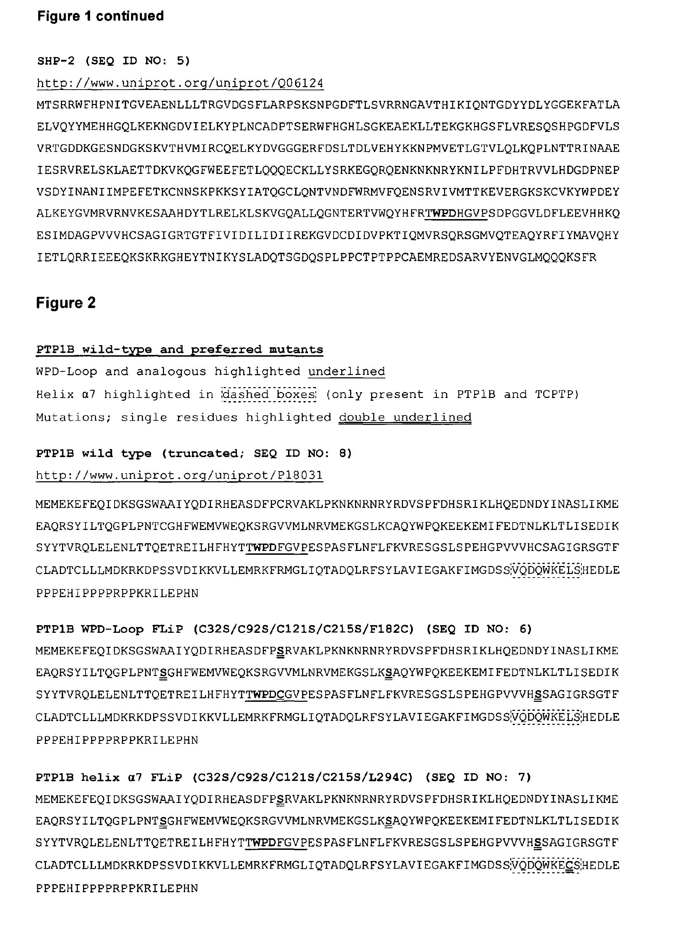

The WPD loop can range between seven and ten residues in length. Typically, a length of eight or nine residues is observed; see, for example, the attached Figures 1 and 2. This loop comprises the conserved three amino acid motif WPD. As is apparent from Figure 1 enclosed herewith, the three amino acid motif WPD is not always strictly conserved. For example, the sequence FPD may also take its place. With the information provided on the location of the WPD loop within the primary structure of the protein phosphatases given in Figure 1, the skilled person can determine without further ado, for example by performing pairwise or multiple sequence alignments, the presence and location of a WPD loop in any other protein phosphatase, also noting that such other phosphatase may present a conserved variant of the WPD sequence such as FPD. An example of a multiple sequence alignment can be found in Figure 1 of Andersen et al. (Molecular and Cellular Biology, 21, 7117-7136, 2001). A structural analysis of the classical human protein tyrosine phosphatome can be found, for example, in Barr et al. (Cell 136, 352-363, 2009). A diagram of the three dimensional structure of PTP1B, a preferred wild-type protein phosphatase according to the present invention, can be found in Figure 2 of Kamerlin et al. (BBRC, 356, 1011-1016, 2007). For the purposes of the present invention, the boundaries of the WPD loop are those as apparent from Figures 1 and 2 enclosed herewith. In particular, the WPD loop of PTP1B (SEQ ID NO: 1), TCTPT (SEQ ID NO: 2), MptbB (SEQ ID NO: 3), SHP-1 (SEQ ID NO: 4) and SHP-2 (SEQ ID NO: 5) is formed by residues 178 to 185, 179 to 186, 77 to 85, 416 to 423, and 426 to 433, respectively; see the sub-sequences highlighted in light grey in Figure 1.

The $\alpha 7$ helix is present, for example, in PTP1 B and TCPTP; see residues 287 to 295 and 285 to 293 in the corresponding sequences as shown in Figure 1, said ranges being highlighted in dark gray. A diagram showing the three dimensional structure of PTP1 B, the $\alpha 7$ helix being indicated, can be found in Figure 2 of Kamerlin et al. (loc.cit.). The residue ranges constituting the $\alpha 7$ helix in any other protein phosphatase can be determined by the skilled person without further ado by performing pairwise sequence alignment of the sequence of any one of PTP1B (SEQ ID NO: 1) or TCTPT (SEQ ID NO: 2) with the respective protein phosphatase at issue, or by performing a multiple sequence alignment comprising the sequences of both SEQ ID NOs: 1 and 2 as well as of one or more further protein phosphatases, wherein the location of the $\alpha 7$ helix in said further phosphatase(s) is to be determined.

A protein phosphatase according to the main embodiment containing exactly one label in the WPD loop constitutes a vehicle suitable for an assay for active site-binders or active site inhibitors. To explain further, the WPD loop is adjacent to the active site and known to exist in at least two distinct conformations, only one of which is catalytically competent. Any change of conformation of the WPD loop is reflected in the signal generated by the label present in the protein phosphatase according to (a)(ii) of the main embodiment.

The C-terminal helix, also referred to as $\alpha 7$ helix herein, is one of the secondary structure elements forming an allosteric binding pocket; see, for example, Wiesmann et al. (Nature, structural & molecular biology, 11, 730-737, 2004). The residues forming said allosteric pocket are, generally speaking, quite variable. For example, and as is stated herein above, the allosteric pocket of PTP1B contains an F residue at position 280 at the C-terminal end of the $\alpha 6$ helix which F residue is absent in TCPTP. As a consequence, targeting the allosteric pocket is an approach being particularly suitable for the identification of specific binders, modulators or inhibitors. By placing a label in the $\alpha 7$ helix, any conformational change arising from the binding of a compound to said pocket can be specifically and sensitively detected. Binding of ligands to the allosteric site prevents the WPD loop from adopting a catalytically competent conformation.

The $\alpha 7$ helix is just one of the structural elements forming the described allosteric binding pocket. While the $\alpha 7$ helix is a preferred region for introducing a label according to the present invention, it is also deliberately envisaged to alternatively introduce said label in any of the other structural elements forming said allosteric binding pocket. These other structural elements include the mentioned $\alpha 6$ helix. Provided with the teaching of the present application, the skilled person can identify further structural elements forming the allosteric binding pocket, said further structural elements being also - albeit less preferred - targets for labeling.

The protein phosphatase according to the present invention which is labeled in a WPD-loop may also be used for the identification of specific inhibitors despite the higher degree of conservation in the WPD-loop as compared to the $\alpha 7$ helix and other structural elements forming the mentioned allosteric binding pocket. To explain further, if inhibition of a given first protein phosphatase (such as PTP1 B) is desired, wherein a given second protein phosphatase (such as TCTPT) should not be significantly inhibited, one may select the following procedure. Screens are conducted with both protein phosphatases, each of them being labeled in the WPD-loop, preferably at corresponding, i.e. aligning, positions and accordingly constituting protein phosphatases according to the present invention. When evaluating the results of the screens, preference is given to those compounds which inhibit said first protein phosphatase to a higher degree as compared to said second protein phosphatase.

Preferred is a protein phosphatase which is a protein tyrosine phosphatase, and said structural element is the WPD loop. Similarly, it is preferred that said protein phosphatase is PTP1B or TCPTP, and said structural element is the $\alpha 7$ helix, also referred to as C-terminal helix.

On the other hand, it is deliberately envisaged to label also structural elements within a protein phosphatase which are adjacent to those mentioned above, i.e. adjacent to either the WPD-loop or the $\alpha 7$ helix. By adopting such a strategy, it is expected to be possible to monitor any movements of the WPD-loop or the $\alpha 7$ helix, any such movement being indicative of the binding of a candidate modulator or inhibitor.

Both within the $\alpha 7$ helix and the WPD-loop, preference is given to solvent exposed amino acids. Using PTP1B (SEQ ID NO: 1) as an example, preferred residues for introduction of a label, for example by replacement with Cys and derivatization thereof with a fluorophor, are the following amino acids: Q288, D289, Q290 and E293. These four residues are located within the $\alpha 7$ helix of PTP1B; see Figure 1 as enclosed herewith. As regards the WPD-loop, preference is given to F182 in the sequence of SEQ ID NO: 1. Corresponding positions in any other protein phosphatase are also envisaged as targets for labeling, wherein the term "corresponding" refers to positions aligning with the above mentioned positions of SEQ ID NO: 1 in a pairwise or multiple sequence alignment of protein phosphatases, said alignment including the sequence of SEQ ID NO: 1 and the sequence of a protein phosphatase, wherein preferred positions for labeling in the latter protein phosphatase are to be determined.

The term "label" is well-known in the art. In the present case, the label according to the invention is to be selected from a fluorophore, a spin label and an isotope label which are discussed in more detail below. Importantly, the label according to the invention is a moiety which does not occur in the wild type form of said protein phosphatase. For example, a fluorophore as comprised in a naturally occurring amino acid is not a label according to the invention. Similarly, an amino acid containing a 13C nucleotide is not a label according to the invention if the frequency of 13C at the given position merely arises from the natural distribution of isotopes.

The term "wild type form" as used herein is meant to distinguish naturally occurring protein phosphatases from (i) protein phosphatases which have sequences that are modified in accordance with the present invention for the purpose of labeling and (ii) protein phosphatases containing a label in said structural element according to the invention. Accordingly, the term "wild type form" includes naturally occurring mutants such as, for example, disease-relevant mutants, provided that those mutants are neither labeled in accordance with the invention nor have a sequence which is modified for the purpose of labeling. It is understood that in such naturally occurring mutant forms the mutated position is generally irrelevant for and not useful for labeling.

In other words, a given protein phosphatase may occur in at least three different forms. First, there is the wild type form of the protein phosphatase which, for the purpose of labeling, might require certain modifications of the sequence such as the introduction of a site amenable to labeling. Such modifications are further detailed below. Another type of modification which might be necessary is the removal of one or more sites where labeling is not desired. In either of the two cases, a difference in sequence will arise between the wild type form of the protein phosphatase and the protein phosphatase which is amenable to labeling. Upon labeling, which may be achieved by reacting with reactive forms of fluorophores, spin labels and isotope labels, a further form is obtained which is the protein phosphatase according to the invention. Said protein phosphatase according to the invention differs from the wild type form thereof in that it is labeled, and, if the sequence of the wild type form was not suitable for labeling, by one or more sequence modifications.

It is understood that the term "protein phosphatase" as used herein also refers to fragments of naturally occurring sequences, said fragments optionally being further derivatized for the purpose of labeling, if necessary, as defined herein above. It is preferred that such fragments contain all structural elements necessary for maintaining the correct fold of the protein phosphatase domain. Presence of the correct fold can be determined, for example, in an activity assay. An example of an activity assay is a test for the dephosphorylating activity of the protein phosphatase. Suitable assays are described, for example, in Vintonyak et al. (Tetrahedron 67, 6713-6729, 2011).

An example of such a fragment is the PTP1 B fragment the sequence of which is set forth in SEQ ID NO: 8. SEQ ID NO: 8 is a C-terminally truncated form of SEQ ID NO: 1, SEQ ID NO: 1 being the wild-type PTP1 B sequence.

The term "fluorophore" is well-known in the art and denotes a molecule or functional group within a molecule which absorbs energy such as a photon of a specific wavelength and emits energy, i.e. light at a different (but equally specific) wavelength immediately upon absorbance (unlike the case in phosphorescence) without the involvement of a chemical reaction (as the case in bioluminescence). This type of emission is known as fluorescence. Usually the wavelength of the absorbed photon is in the ultraviolet range but can reach also into the visible and infrared range. The wavelength of the emitted light is shifted towards longer wavelengths as compared to the absorption wavelength and is usually in the visible range. The amount and wavelength of the emitted energy depend primarily on the properties of the fluorophore but is also influenced by the chemical environment surrounding the fluorophore. Fluorophores can be sensitive to changes in their environment. Preferred fluorophores are sensitive to the environment. This includes changes in the polarity, which occur when the conformation of the molecule to which they are attached changes. Fluorescence occurs when a molecule relaxes to its ground state after being electronically excited which, for commonly used fluorescent compounds that emit photons with energies from the UV to near infrared, typically happens in the range of between 0.5 and 20 nanoseconds. Fluorophores typically exhibit changes in intensity and/or a shift in the wavelength of maximum emission depending on the polarity of the surrounding chemical environment. In the present case, the chemical environment is formed by the structural elements of the protein phosphatase, in particular those structural elements of the protein phosphatase which are in close spatial proximity of the label, as well as by surrounding molecules including solvent molecules.

Preferred fluorophores are 6-acryloyl-2-dimethylaminonaphthalene (Acrylodan), 6-bromoacetyl-2-dimethylamino-naphthalenebadan (Badan), 2-(4'-(iodoacetamido)anilino)naphthalene-6-sulfonic acid, sodium salt (IAANS), 2-(4'-maleimidylanilino)naphthalene-6-sulfonic acid, sodium salt (MIANS), 5-((((2-iodoacetyl)amino)ethyl)amino)naphthalene-1-sulfonic acid (1,5-IAEDANS) and 5-dimethylaminonaphthalene-1-sulfonyl aziridine (dansyl aziridine) or a derivative thereof.

Other fluorophores which may be used are coumarin-based compounds, benzoxadiazole-based compounds, dapoxyl-based compounds, biocytin-based compounds, fluorescein, sulfonated rhodamine-based compounds such as AlexaFluor dyes (Molecular Probes), Atto fluorophores (Atto Technology) or Lucifer Yellow. Coumarin-based fluorophores are moderately sensitive to environment and 7-diethylamino-3-(4'-maleimidylphenyl)-4-methylcoumarin (CPM) is an example. Benzoxadiazole fluorophores are also commonly used for forming protein-fluorophore conjugates and have a strong environmental dependence with 7-fluorobenz-2-oxa-1,3-diazole-4-sulfonamide (ABD-F) and N-((2-(iodoacetoxy)ethyl)-N-methyl)amino-7-nitrobenz-2-oxa-1,3-diazole ester (IANBD) as examples. PyMPO maleimide (for thiols) or succinimide ester (for amines) and various other dapoxyl dyes have good absorptivity and exceptionally high environmental sensitivity. Examples are 1-(2-maleimidylethyl)-4-(5-(4-methoxyphenyl)oxazol-2-yl)pyridinium methanesulfonate (PyMPO-maleimide), 1-(3-(succinimidyloxycarbonyl)benzyl)-4-(5-(4-methoxyphenyl)oxazol-2-yl) pyridinium bromide (PyMPO-succinimidyl ester) and Dapoxyl (2-bromoacetamidoethyl) sulphonamide.

Recording the fluorescence emission signal at one or more wavelengths or a spectrum is usually accomplished using a fluorescence spectrometer or fluorimeter. Fluorescence spectroscopy or fluorimetry or spectrofluorimetry is a type of electromagnetic spectroscopy which analyzes fluorescence or other light emitted from a sample. It involves using a beam of light, usually ultraviolet light, that excites the electrons in certain molecules (fluorophores) and causes them to emit light of a lower energy upon relaxation, typically, but not necessarily, visible light.

Two general types of instruments exist which can both be employed in the method of the invention: Filter fluorimeters use filters to isolate the incident light and fluorescent light, whereas spectrofluorimeters use diffraction grating monochromators to isolate the incident light and fluorescent light. Both types utilize the following scheme: The light from an excitation source passes through a filter or monochromator and strikes the sample. A proportion of the incident light is absorbed by the sample, and some of the molecules in the sample fluoresce. The fluorescent light is emitted in all directions. Some of this fluorescent light passes through a second filter or monochromator and reaches a detector, which is usually placed at 90° to the incident light beam to minimize the risk of transmitted or reflected incident light reaching the detector. Various light sources may be used as excitation sources, including lasers, photodiodes, and lamps; xenon and mercury vapor lamps in particular. The detector can either be single-channeled or multi-channeled. The single-channeled detector can only detect the intensity of one wavelength at a time, while the multi-channeled detects the intensity at all wavelengths simultaneously, making the emission monochromator or filter unnecessary. The different types of detectors have both advantages and disadvantages. The most versatile fluorimeters with dual monochromators and a continuous excitation light source can record both an excitation spectrum and a fluorescence spectrum. When measuring fluorescence spectra, the wavelength of the excitation light is kept constant, preferably at a wavelength of high absorption, and the emission monochromator scans the spectrum. For measuring excitation spectra, the wavelength passing though the emission filter or monochromator is kept constant and the excitation monochromator is scanning. The excitation spectrum generally is identical to the absorption spectrum as the fluorescence intensity is proportional to the absorption (for reviews see Rendell, 1987; Sharma and Schulman,1999; Gauglitz and Vo-Dinh, 2003; Lakowicz, 1999).

The term "spin label" (SL) denotes a molecule, generally an organic molecule, which possesses an unpaired electron, usually on a nitrogen atom. Spin labels are used as tools for probing proteins using electron paramagnetic resonance (EPR) spectroscopy. The site-directed spin labeling (SDSL) technique allows one to monitor the conformation and dynamics of a protein. In such examinations, amino acid-specific SLs can be used.

In a further preferred embodiment, the thiol-reactive spin-label is a nitroxide radical. A preferred method for site-specifically labeling protein sequences with a spin-label is the reaction between methanethiosulfonate spin label and cysteine, to give the spin-labeled cysteine side chain, CYS-SL:

$$MeS(O)2SSR + R'SH \rightarrow R'SSR + MeS(O)2SH$$

where R is the nitroxide group and R'SH is a protein with a cysteine sulfhydryl, and R'SSR is the spin-labeled protein. The cysteines for labeling are placed in the desired sequence position either through solid-phase techniques or through standard recombinant DNA techniques.

Site-directed spin labeling is a technique for investigating protein local dynamics using electron spin resonance. SDSL is based on the specific reaction of spin labels carrying a reactive group with amino acids. A spin label built in protein structures can be detected by EPR spectroscopy. In SDSL, sites for attachment of spin labels such as thiol or side chain amino groups, if not naturally present, are introduced into recombinantly expressed proteins, for example by site-directed mutagenesis. In other words, by the above techniques, spin labels can be introduced into a protein phosphatase such that said protein phosphatase is specifically labeled, preferably only at the desired position. Reactive functional groups contained within the spin label determine their specificity. At neutral pH, protein thiol groups specifically react with functional groups such as methanethiosulfonate, maleimide and iodoacetamide, creating a covalent bond with the amino acid cysteine. It is understood that the spin label according to the main embodiment no longer contains a reactive functional group. As required by the invention, the spin label (already) is connected to an amino acid of the protein phosphatase via a covalent bond. Spin labels are unique molecular reporters in that they are paramagnetic, i.e. they contain an unpaired electron. For example, nitroxide spin labels are widely used for the study of macromolecular structure and dynamics because of their stability and simple EPR signal. The nitroxyl radical (N-O) is usually incorporated into a heterocyclic ring such as pyrrolidine, and the unpaired electron is predominantly localized to the N-O bond. Once incorporated into the protein, a spin label's motions are dictated by its local environment. Because spin labels are exquisitely sensitive to motion, this has profound effects on the EPR spectrum of the spin-label attached to the protein.

The signal arising from an unpaired electron can provide information about the motion, distance, and orientation of unpaired electrons in the sample with respect to each other and to the external magnetic field. For molecules free to move in solution, EPR works on a much faster time-scale than NMR (Nuclear Magnetic Resonance) spectroscopy, and accordingly can reveal details of much faster molecular motions, i.e. nanoseconds as opposed to microseconds for NMR. The gyromagnetic ratio of the electron is orders of magnitude larger than of nuclei commonly used in NMR, and so the technique is more sensitive than NMR.

Electron paramagnetic resonance (EPR) or electron spin resonance (ESR) spectroscopy is a technique for studying chemical species that have one or more unpaired electrons, such as organic and inorganic free radicals or inorganic complexes possessing a transition metal ion. The basic physical concepts of EPR are analogous to those of nuclear magnetic resonance (NMR), but it is electron spins that are excited instead of spins of atomic nuclei. Because most stable molecules have all their electrons paired, the EPR technique is less widely used than NMR. However, this limitation to paramagnetic species also means that the EPR technique is one of great specificity, since ordinary chemical solvents and matrices do not give rise to EPR spectra.

An isotope label according to the invention is, as defined above, a moiety comprising one or more atoms which are isotopes with non-zero nuclear spin, wherein said isotopes with non-zero nuclear spin are enriched as compared to the natural occurrence thereof. Isotopes are nuclides with the same atomic number but different mass number. The most common isotopes with non-zero nuclear spin are 1H, 2D, 15N, 13C, and 31 P. Preferred isotopes are 13C and 15N.

According to the present invention, two distinct types of isotope labels are envisaged. First, the isotope label, similar to fluorophores and spin labels, is a chemical moiety which is attached to a functional group of an amino acid of the protein phosphatase. Any moiety which is enriched with regard to isotopes having non-zero nuclear spin is envisaged. For the purpose of attaching such isotope labels, reactive groups as described above in relation to fluorophores may be used. Alternatively, the isotope label may be an amino acid residue present in the protein phosphatase, wherein the occurrence of nuclei with non-zero nuclear spin is elevated as compared to the natural occurrence in that specific residue. Preferably, all atoms of a given chemical element are replaced by a corresponding isotope with non-zero nuclear spin.

Nuclear magnetic resonance (NMR) is a physical phenomenon based upon the quantum mechanical magnetic properties of the nucleus of an atom. All nuclei that contain odd numbers of protons or neutrons have an intrinsic magnetic moment and angular momentum. The most commonly measured nuclei are hydrogen (1H) (the most receptive isotope at natural abundance) and carbon (13C), although nuclei from isotopes of many other elements (e.g. 113Cd, 15N, 14N, 19F, 31 P, 170, 29Si, 10B, 11 B, 23Na, 35Cl, 195Pt) can also be observed. NMR resonant frequencies for a particular substance are directly proportional to the strength of the applied magnetic field, in accordance with the equation for the Larmor precession frequency. NMR measures magnetic nuclei by aligning them with an applied constant magnetic field and perturbing this alignment using an alternating magnetic field, those fields being orthogonal. The resulting response to the perturbing magnetic field is the phenomenon that is exploited in NMR spectroscopy and magnetic resonance imaging, which use very powerful applied magnetic fields in order to achieve high spectral resolution, details of which are described by the chemical shift and the Zeeman Effect.

The term "amino acids" has its established meaning and refers to organic molecules that have a carboxylic and an amino functional group. They are the essential building blocks of proteins. Examples of amino acids having a free thiol group are cysteine, belonging to the 20 proteinogenic amino acids, and acetyl-cysteine being a non-standard amino acid rarely occurring in natural amino acid sequences. Proteinogenic amino acids having a free side chain amino group are lysine, histidine or arginine and amino acids being aromatic amines, such as tryptophan. Pyrrolysine, 5-hydroxylysine or o-aminotyrosine are non-standard amino acids having a free side chain amino group. The amino acids asparagine and glutamine instead contain an amide group and are not suitable in the present invention as they are not reactive to labeling agents and are thus excluded.

Tryptophan is an aromatic amino acid having an amino group in its indole ring. Aromatic amines are weak bases and thus unprotonated at pH 7. However, they can be modified using a reactive reagent such as an isothiocyanate, or sulfonyl chloride.

In a preferred embodiment, said label in said structural element is the only fluorophore, spin label or isotope label present in said protein phosphatase. While the main embodiment allows for one or more fluorophores, spin labels and/or isotope labels outside said structural element, this preferred embodiment specifies that the single label present in said structural element is the only fluorophore, spin label or isotope label throughout the protein phosphatase.

In a further preferred embodiment, the amino acid sequence of said structural element carrying said label is (a) the amino acid sequence of the wild type form of said protein phosphatase; (b) differs from the amino acid sequence of said wild type form by the presence of one amino acid residue containing a thiol group or side chain amino group at a position where the amino acid sequence of the wild type does not contain such a residue; or (c) differs from the amino acid sequence of the wild type form by the absence of one or more amino acid residues containing a thiol group or side chain amino group, provided that exactly one amino acid residue or at least one amino acid residue containing a thiol group or side chain amino group is present in said structural element carrying said label.

Depending on the amino acid residues being present in said structural element of the wild type form of a given protein phosphatase, either the wild type form as such may be amenable to labeling, or certain sequence modifications may be required in order to introduce an amino acid residue amenable to labeling. In either case, said structural element contains at least one, preferably exactly one amino acid residue amenable to labeling, i.e., at least one or exactly one amino acid residue containing a thiol group or a side chain amino group. The recited presence of one amino acid residue according to option (b) of this embodiment may be achieved either by substitution or insertion of said one amino acid residue. Similarly, the absence of one or more amino acid residues according to option (c) may be achieved by substitution of more or more amino acid residues or deletion of one or more amino acid residues, in either case resulting in the removal of one or more amino acid residues containing a thiol group or side chain amino group. For any of these sequence modifications, means and methods are available at the skilled persons disposal. Such methods include site-directed mutagenesis as well as other recombinant synthetic or semi-synthetic techniques. In case any of the above described non-standard amino acids is to be introduced into the phosphatase, an amino acid stretch containing said amino acid may be chemically synthesized and then connected to the remaining part(s) of the phosphatase which may have been produced recombinantly or synthetically. Alternatively, phosphatases expressed and designed to incorporate a specialized non-standard amino acid at the desired position for subsequent labeling may be produced recombinantly by applying an altered genetic code (see e. g. Liu and Schultz, 2010). Preferred means for achieving presence of an amino acid according to option (b) are substitution, and the preferred means for achieving absence of one or more amino acid residues according to option (c) is substitution as well.

The term "delete" refers to excision of an amino acid without replacing it with another amino acid whereas the term "replace" refers to the substitution of an amino acid with another amino acid.

Preferably, amino acid replacements should be conservative. For cysteine, this means that it is preferably replaced with serine. In general, replacements of amino acids with different amino acids may be evaluated in view of whether they are conservative using the PAM250 Scoring matrix. The matrix is frequently used to score aligned peptide sequences to determine the similarity of those sequences (Pearson, 1990).

In a further preferred embodiment, (a) the amino acid sequence of said phosphatase outside said structural element is that of the wild type form; or (b) one, more or all amino acid residues containing a thiol group or side chain amino group and outside said structural element are deleted or replaced with amino acid residues which do not contain a thiol group or side chain amino group. While the preceding preferred embodiment relates to the sequence of the structural element, the present preferred embodiment relates to the sequence of the remainder of the phosphatase according to the invention. According to option (b), those amino acids which are potentially amenable to labeling (amino acid residues containing a thiol group or side chain amino group) are deleted or replaced with amino acid residues which are not amenable to labeling. This is a further preferred measure which ensures that only a single site, said single site being located in the said structural element, is labeled in those cases wherein the labeled form of the phosphatases obtained by contacting the wild type form of the phosphatase in its entirety with a labeling agent under suitable conditions. This approach is exemplified by the PTP1B mutants of SEQ ID NOs: 6 and 7. As stated above, means and methods for targeted sequence modifications, including said directed mutagenesis, are available to the skilled person.

In a preferred embodiment, said amino acid residues containing a thiol group or a side chain amino group and outside the said structural element according to (b) are solvent-exposed. As is known in the art, solvent-exposed amino acids, to the extent they contain suitable functional groups (thiol or side chain amino groups), are more likely to react with a reactive agent. Therefore, it is preferred that solvent-exposed amino acids, more specifically only solvent-exposed amino acids containing a thiol group or a side chain amino group and outside the said structural element are deleted or replaced as defined according to (b) of the preceding embodiment.

The term "solvent-exposed" refers to the position of an amino acid in the context of the three dimensional structure of the protein of which it is a part. Amino acids buried within the protein body are completely surrounded by other amino acids and thus do not have contact with the solvent. In contrast, solvent-exposed amino acids are partially or fully exposed to the surrounding solvent and are thus accessible to chemicals potentially able to modify them. This applies e.g. to thiol- or amino-reactive labels used in the present invention which can react with solvent-exposed amino acids having a free thiol- or amino-group.

The determination of solvent-exposed amino acid residues can be performed by a skilled person without further ado. For example, the wild type form of the phosphatase can be contacted with agents reacting with thiol groups and or side chain amino groups. Subsequently, the labeled positions may be determined, thereby determining the solvent-exposed amino acid residues containing a thiol group or side chain amino group. Alternatively or in addition, computational means may be used. Such computational means make us of the three-dimensional structure of the phosphatase, said three-dimensional structure being either experimentally determined (such as by X-ray crystallography or NMR), or predicted by methods such as homology modeling. Provided with the coordinates of the three-dimensional structure, the skilled person may use established bioinformatic tools which determine solvent exposure of each individual atom as well as of residues in their entirety.

In a further preferred embodiment, said phosphatase differs from the amino acid sequence of said wild type form by the presence of exactly one amino acid residue containing a thiol group or side chain amino group at a position where the amino acid sequence of the wild type does not contain such a residue. This embodiment relates to a labeled phosphatase according to the present invention which has the amino acid sequence of the wild type form outside the said structural element, and exactly one amino acid residue amenable to labeling, i.e., an amino acid residue containing a thiol group or a side chain amino group within the said structural element, wherein said amino acid residue amenable to labeling is not present in the sequence of the said structural element in the wild type form of said phosphatase.

In a more preferred embodiment, said exactly one amino acid residue is a cysteine replacing an amino acid residue of said wild type form.

In a further preferred embodiment, the amino acid sequence of said protein phosphatase or of the wild type form thereof is the amino acid sequence of PTP1 B, TCPTP, MptpB, SHP-1 or SHP-2.

PTP1B is an abbreviation for tyrosine protein phosphatase non-receptor type 1, also known as protein tyrosine phosphatase 1 B (EC 3.1.3.48). TCPTP is an abbreviation for tyrosine phosphatase non-receptor type 2, also known as T-cell protein tyrosine phosphatase (EC 3.1.3.48). MptpB is an abbreviation for *Mycobacterium tuberculosis* phosphotyrosine protein phosphatase ptpB (EC 3.1.3.48). SHP-1 is an abbreviation for tyrosine protein phosphatase non-receptor type 6, also known as hematopoietic cell protein tyrosine phosphatase or protein tyrosine phosphatase 1C (EC 3.1.3.48). SHP-2 is an abbreviation for tyrosine protein phosphatase non-receptor type 11, also known as protein tyrosine phosphatase 1 D or protein tyrosine phosphatase 2C (EC 3.1.3.48).

With the exception of the above described mycobacterial protein tyrosine phosphatase, it is preferred that said protein tyrosine phosphatases are of human origin. The sequences of PTP1 B, TCPTP, SHP-1 and SHP-2 as shown in Figure 1 are of human origin. Accordingly, preferred sequences of PTP1B, TCPTP, MptpB, SHP-1 and SHP-2 are those of SEQ ID NOs: 1, 2, 3, 4 and 5, respectively.

Particularly preferred is the sequence of SEQ ID NO: 1, wherein position 182 has been replaced with a cysteine residue, or wherein position 294 has been replaced with a cysteine residue.

In a yet more preferred embodiment, the amino acid sequence of said protein phosphatase is the sequence set forth in SEQ ID NO: 6 or 7. In these two sequences, cysteine residues other than those intended to be labeled have been replaced with serine residues. While a replacement with serine is particularly preferred, it is at the same time envisaged to replace cysteines, the labeling of which is not desirable with any other residues, for example alanine.

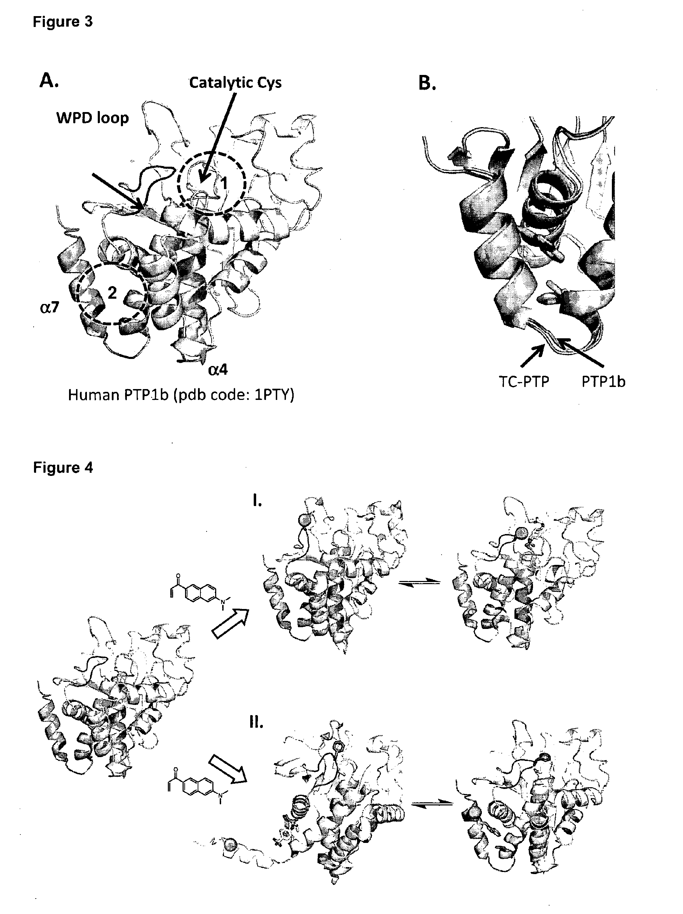

In the most preferred embodiment, there is exactly one label in said structural element, said label being acrylodan covalently bound to the thiol group of the cysteine residue at position 182 of SEQ ID NO: 6 or at position 294 or SEQ ID NO: 7. The subject-matter of this last embodiment is further described in the enclosed example and shown in the attached Figure 4.

The present invention furthermore provides a method of screening for ligands of the wild type form of the protein phosphatase defined in any of the preceding embodiments, said method comprising: (a) contacting the protein phosphatase of the preceding embodiments with a candidate compound; and (b) recording the fluorescence emission signal, the EPR signal or the NMR signal of said label of said protein phosphatase of any one of the preceding embodiments, wherein a difference in signal as compared to the signal in absence of said candidate compound is indicative of said candidate compound being a ligand of the wild type form of the protein phosphatase defined in any one of the preceding embodiments.

The term "ligand" has the meaning as established in the art. It refers to any compound which directly interacts or binds to a protein phosphatase. Preferably, the equilibrium binding constant is in the millimolar, nanomolar, picomolar or below range. It is understood that in the course of the screening typically one or more of the tested compounds is discarded because it does not exhibit any measurable binding.

Said contacting according to (a) occurs under conditions which allow binding of a ligand known to be capable of binding to said protein phosphatase. The skilled person, being acquainted with the handling of protein phosphatase as well as the design of assays using protein phosphatase as a target, can establish such suitable conditions without further ado. Such conditions include buffered solutions.

Depending on whether the label present in the protein phosphatase according to the invention is a fluorophore, spin label or an isotope label, step (b) of the method according to the invention provides for recording the fluorescence emission signal, recording the EPR signal, or recording the NMR signal, respectively.

It is understood that the term "signal" embraces a measurement at a single wavelength as well as a spectral scan. Examples of a fluorescent spectrum, and how this spectrum is subjected to change upon addition of a ligand, is shown in the Examples as enclosed herewith. In case it is not yet known for a given labeled protein phosphatase where in the spectrum the maximum change in signal occurs upon ligand binding, it will in general be advisable to perform a spectral scan in order to determine the wavelength of maximum change upon ligand binding. If this is already known, performing the measurement at a single wavelength, preferably at the wave length of maximum change, is sufficient. A "difference in signal" refers to a change of the entire fluorescence emission spectrum, EPR spectrum or NMR spectrum, a shift of the maximum emission wavelength, and/or a change of emission at the given wavelength.

The present invention furthermore provides a method of screening for inhibitors of the wild type form of the protein phosphatase defined above, said method comprising the method of screening for ligands as defined above and (c) determining the activity of said protein phosphatase defined herein above or of said wild type form thereof in the presence of said candidate compound, wherein a decrease in activity as compared to the absence of said candidate compound is indicative of said candidate compound being an inhibitor of the wild type form of the protein phosphatase defined above.

This aspect of the present invention provides for screening for specific types of ligands which are inhibitors of protein phosphatase activity. Accordingly, the method includes the further step of monitoring the activity of the protein phosphatase. The activity of the protein phosphatase may be the biochemical activity, said biochemical activity embracing dephosphorylating activity with regard to the target of the protein phosphatase. Alternatively or in addition, the activity at a cellular level may be determined. This includes readouts not at the level of the protein phosphatase itself, but instead, for example, of an entire pathway comprising the protein phosphatase at issue. If the protein phosphatase is disease-relevant, disease models may be used for monitoring activity. For example, PTP1B is considered a drug target for the treatment of type II diabetes and obesity. Accordingly, established models of type II diabetes or obesity may be used in an activity assay monitoring protein phosphatase activity in accordance with this aspect of the present invention. These and further protein phosphatase activity assays are within the skills of the skilled person.

Protein phosphatase inhibitors are substances capable of inhibiting the activity of protein phosphatase. They can more specifically inhibit the action of a single protein phosphatase. Depending on the structural element chosen for labeling, the inhibitors identifiable by the method of screening for inhibitors according to the invention may either be active site inhibitors or allosteric inhibitors. More specifically, if the WPD loop is chosen as a structural element for labeling, the inhibitors to be identified are likely active site inhibitors. On the other hand, if the $\alpha7$ helix is chosen, the inhibitors to be identified are likely allosteric inhibitors.

A candidate inhibitor may belong to different classes of compounds such as small organic or inorganic molecules, proteins or peptides, nucleic acids such as DNA or RNA. Such compounds can be present in molecule libraries or designed from scratch.

Small molecules according to the present invention comprise molecules with a molecular weight of up to 2000 Da, preferably up to 1500 Da, more preferably up to 1000 Da and most preferably up to 500 Da.

Preferably, the method of screening according to the invention is effected in high-throughput format. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact with test compounds, in this case putative inhibitors, with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits inhibitory activity, said mixture of test inhibitors may be de-convoluted to identify the one or more test inhibitors in said mixture giving rise to said activity.

Alternatively, only one test inhibitor may be added to a well, wherein each test inhibitor is applied in different concentrations. For example, the test inhibitor may be tested in two, three or four wells in different concentrations. In this initial screening, the concentrations may cover a broad range, e.g. from 10 nM to 10 $\mu$M. The initial screening serves to find hits, i.e. test inhibitors exerting inhibiting activity at at least one concentration, preferably two, more preferably all concentrations applied, wherein the hit is more promising if the concentration at which an inhibitory activity can be detected is in the lower range. This alternative serves as one preferred embodiment in accordance with the invention.

In a further aspect, the present invention provides a method of quantifying ligand or inhibitor binding to a protein phosphatase, said method comprising (a) (i) contacting a protein phosphatase according to the invention with different concentrations of a ligand or inhibitor; and/or (ii) contacting a complex, the complex comprising or consisting of a protein phosphatase according to the invention and a ligand or inhibitor, with different concentrations of unlabeled, but otherwise the same protein phosphatase or of the wild type form of said protein phosphatase; (b) recording the signal emitted by the label of said protein phosphatase according to the invention at a given time point after said contacting and for each concentration of ligand or inhibitor according to (a)(i) and/or for each concentration of unlabeled or wild type form protein phosphatase according to (a)(ii); and (c) determining the dissociation and/or association constant of said complex from the concentration dependence of said signal recorded according to (b).

This aspect of the present invention refers to so-called endpoint measurements. This is reflected by the feature "at a given time point after said contacting" as required by step (b) of the method. This method allows one to determine the equilibrium dissociation constant $K_d$ of the complex formed by the protein phosphatase and the ligand. The association

constant $K_a$ is related to the dissociation constant according to $K_a=1/K_d$.

The term "quantifying ligand or inhibitor binding to a protein phosphatase" refers to the determination of kinetic and/or thermodynamic parameters governing the interaction between the protein phosphatase and the ligand or inhibitor.

As previously discussed in relation to other embodiments, said contacting occurs under conditions which allow binding of a known ligand to a given protein phosphatase.

Suitable time points after said contacting can be determined by the skilled person without further ado.

The end point signal, when plotted as a function of the logarithm of the concentration according to (a)(i) or (a)(ii), usually has sigmoidal shape. The concentration corresponding to the inflection point of the sigmoidal curve corresponds to the value of $K_d$. Measuring the same samples over time and making several such curves will reveal the time needed. As the compounds bind slowly, the signal changes and the corresponding curve will shift leftward with time until it reaches its final value - this is the true $K_d$ curve for the compound at equilibrium.

In a preferred embodiment, said label is a fluorophore and said signal is the ratio of emission intensities at two different wavelengths, the two wavelengths being local maxima of the emission spectrum. This ratio provides a sensitive measure of changes in the spectrum.

This preferred embodiment is a specific implementation of the general method of quantifying ligand or inhibitor binding according to the present invention, wherein said preferred embodiment relates to those labeled protein phosphatase wherein the label is a fluorophore.

Alternatively, or in addition, further parameters may be determined. These parameters include the ratio of the normalized intensity change to the average intensity change ($\Delta I_{std}$), the maximum standard intensity change ($\Delta R_{max}$) as well as the Z-factor. These quantities are defined and discussed below. A labeled protein phosphatase according to the invention, particularly a fluorescently labeled protein phosphatase according to the invention is considered particularly suitable for the screening of the protein phosphatase inhibitors if ($\Delta I_{std}$) is > 0.25, and/or ($\Delta R_{max}$) is > 0.75 and the Z-factor is > 0.5.

$\Delta I_{std}$ is the ratio of normalized intensity change to average intensity of the fluorescence emission. According to de Lorimier et al. (2002), $\Delta I_{std}$ is one of the most important criteria for characterizing a fluorescent protein conjugate as suitable for sensitive fluorescence spectroscopy. Ideally, the $\Delta I_{std}$ should have a value > 0.25 and is calculated by:

$$\Delta I_{std} = \left| \frac{2(I_1(\lambda_{std}) - I_2(\lambda_{std}))}{I_1(\lambda_{std}) + I_2(\lambda_{std})} \right|$$

where $\lambda std = (\lambda max, unbound + \lambda max, saturated)/2$ and I1, I2 are the fluorescence intensities at $\lambda std$ of each spectrum respectively.

$\Delta R_{max}$ is the maximum standard intensity change of the fluorescence emission between saturated and unsaturated protein phosphatase. According to de Lorimier et al. (2002), $\Delta R_{max}$ is another important criteria for characterizing a fluorescent protein conjugate as suitable for sensitive fluorescence spectroscopy. Ideally, the $\Delta R_{max}$ should have a value > 1.25 and is calculated by:

$$\Delta R = \left| \frac{^0A_1}{^0A_2} - \frac{^\infty A_1}{^\infty A_2} \right|$$

where $^OA1$, $^OA2$ are the areas in the absence of ligand, and $^\infty A1$, $^\infty A2$ are the areas in the presence of saturating ligand. A computer program can be used to enumerate $\Delta R$ for all possible pairs of wavelength bands in the two spectra, to identify the optimal sensing condition, defined as the maximum value of $\Delta R$.

The Z-factor is a statistical measure of the quality or power of a high-throughput screening (HTS) assay. In an HTS campaign, large numbers of single measurements of unknown samples are compared to well established positive and negative control samples to determine which, if any, of the single measurements are significantly different from the negative control. Prior to starting a large screening campaign, much work is done to assess the quality of an assay on a smaller scale, and predict if the assay would be useful in a high-throughput setting. The Z-factor predicts if useful data could be expected if the assay were scaled up to millions of samples. The Z- factor is calculated by:

$$Z factor = 1 - \frac{3 \times (\sigma_p + \sigma_n)}{|\mu_p - \mu_n|}$$

wherein both the mean ($\mu$) and standard deviation ($\sigma$) of both the positive (p) and negative (n) controls ($\mu_p, \sigma_p, \mu_n, \sigma_n$, respectively) are taken into account.

The measurement of $\Delta I_{std}$ and $\Delta R_{max}$ as well as the determination of the Z-factor may prove useful in determining whether the label chosen is particularly suitable in the screening for inhibitors. De Lorimier discusses that the measured kinetics and $K_d$ obtained with a fluorescent tagged protein will depend on the protein, the ligand, the chosen labeling site and the fluorophore used. Therefore, the same inhibitor binding to the same labeled protein phosphatase could give different $K_d$ values depending on the label used and its position in the protein. In addition to comparing measured $K_d$ values with known values reported in literature, the determination of the above values might indicate which label should be preferred.

In a further aspect, the present invention provides a method of quantifying ligand or inhibitor binding to a protein phosphatase, said method comprising (a) (i) contacting a protein phosphatase according to the invention with at least one concentration of a ligand or inhibitor; and/or (ii) contacting a complex, the complex comprising or consisting of a protein phosphatase according to the invention and a ligand or inhibitor, with at least one concentration of unlabeled, but otherwise the same protein phosphatase or of the wild type form of said protein phosphatase; (b) recording the signal emitted by the label of said protein phosphatase according to the invention as a function of time upon said contacting according to (a); and (c) (i) determining the rate constant governing the time dependence of said signal according to (b) for (each of) the concentration(s) according to (a)(i) and determining $k_{on}$ therefrom; and/or (ii) determining the rate constant governing the time dependence of said signal according to (b) for (each of) the concentration(s) according to (a)(ii) and determining $k_{off}$ therefrom.

This aspect of the invention relates to real time measurements as indicated by the feature "as a function of time" required by step (b) of the method. The thereby obtained time dependencies allow determination of the kinetic constants $k_{on}$ and $k_{off}$ by established means which is reflected in step (c) of the method according to this aspect of the invention.

In a preferred embodiment, this method of quantifying ligand or inhibitor binding further comprises calculating the dissociation constant and/or association constant from $k_{on}$ and $k_{off}$ as determined in step (c). As is well-known in the art, the association constant $K_a$ is related to the kinetic constants $k_{on}$ and $k_{off}$ as follows: $K_a = k_{on}/k_{off}$.

In chemical kinetics, a rate constant k quantifies the speed of a chemical reaction. For a chemical reaction where substance A and B are reacting to produce C, the reaction rate has the form:

$$\frac{d[C]}{dt} = k(T)[A]^m[B]^n$$

wherein k(T) is the reaction rate constant that depends on temperature, and [A] and [B] are the concentrations of substances A and B, respectively, in moles per volume of solution assuming the reaction is taking place throughout the volume of the solution. The exponents m and n are the orders and depend on the reaction mechanism. They can be determined experimentally.

A single-step reaction can also be described as:

$$\frac{d[C]}{dt} = Ae^{\frac{-E_a}{RT}}[A]^m[B]^n$$

$E_a$ is the activation energy and R is the Gas constant. Since at temperature T the molecules have energies according to a Boltzmann distribution, one can expect the proportion of collisions with energy greater than $E_a$ to vary with $e^{-Ea/RT}$. A is the pre-exponential factor or frequency factor.

$k_{on}$ and $k_{off}$ are constants that describe non-covalent equilibrium binding. When a ligand interacts with a receptor, or when a substrate interacts with an enzyme, the binding follows the law of mass action.

$$R + L \underset{k_{off}}{\overset{k_{on}}{\rightleftharpoons}} RL$$

In this equation R is the concentration of free receptor, L is the concentration of free ligand, and RL is the concentration of receptor-ligand complex. In the case of enzyme kinetics, R is the enzyme, or in this case a protein phosphatase, and L is the substrate, or in this case a candidate or known inhibitor. The association rate constant $k_{on}$ is expressed in units of $M^{-1}sec^{-1}$. The rate of RL formation equals $[R] \times [L] \times k_{on}$. The dissociation rate constant $k_{off}$ is expressed in units of $sec^{-1}$. The rate of RL dissociation equals $[RL] \times k_{off}$. At equilibrium, the backward (dissociation) reaction equals the forward (association) reaction. Binding studies measure specific binding, which is a measure of [RL]. Enzyme kinetic assays assess enzyme velocity, which is proportional to [RL], the concentration of enzyme-substrate complexes.

$$RL = R \cdot L \cdot \frac{k_{on}}{k_{off}}$$

The equilibrium dissociation constant, $K_d$ is expressed in molar units and defined to equal $k_{off}/k_{on}$ to arrive at

$$RL = R \cdot L \cdot \frac{k_{on}}{k_{off}} = \frac{R \cdot L}{K_d}$$

The dissociation constant ($K_d$) corresponds to the concentration of ligand (L) at which the binding site on a particular protein is half occupied, i.e. the concentration of ligand, at which the concentration of protein with ligand bound (RL), equals the concentration of protein with no ligand bound (R). The smaller the dissociation constant, the more tightly bound the ligand is, or the higher the affinity between ligand and protein.

Accordingly, the association constant $K_a$, also called inverse $K_d$, is defined as $1/k_d$. The dissociation constant for a particular ligand-protein interaction can change significantly with solution conditions (e.g. temperature, pH and salt concentration).

In a further aspect, the present invention relates to a method of generating a protein phosphatase suitable for the screening of protein phosphatase inhibitors, said method comprising (a) deleting one or more amino acid residues having a free thiol group or free side chain amino group, if any, present in the wild type form of said protein phosphatase, the labeling of said amino acid residues not being desirable, or replacing them with amino acid residues which do not contain a free thiol or side chain amino group, wherein preferably said amino acid residues the labeling of which is not desirable are solvent-exposed; (b) replacing an amino acid residue in a structural element as defined in the main embodiment in said protein phosphatase with an amino acid residue containing a free thiol or side chain amino group if no amino acid residue containing a free thiol or side chain amino group is or would be present in said structural element after step (a), wherein steps (a) and (b) can be effected in any order; and (c) labeling the protein phosphatase obtained from steps (a) and (b) with a thiol- or amino-reactive fluorophore, thiol- or amino-reactive spin label, and/or thiol- or amino-reactive isotope label.

Steps (a) and (b) of this method serve to ensure that labeling according to step (c) only occurs at the position where labeling is desired.

Labeling according to step (c) makes use of thiol or amino reactive forms of the fluorophore, spin label or isotope label according to the invention.

The term "thiol- or amino-reactive" denotes the property of a compound, e.g. a fluorophore, spin label or isotope label to specifically react with free thiol- or amino groups. This is due to a functional group present in said compound which directs a specific reaction with a thiol or amino group. These functional groups may be coupled to molecules such as fluorophores, spin labels or isotope labels in order to provide labels specific for free thiol- or amino-groups. Examples for thiol-specific compounds are e.g. haloalkyl compounds such as iodoacetamide, maleimides, Hg-Link™ phenylmercury compounds or TS-link™ reagents (both Invitrogen). Haloalkyl compounds react with thiol or amino groups depending on the pH.

The process of labeling involves incubation of the protein phosphatase, optionally modified in accordance with steps (a) and (b) of the method of generating a protein phosphatase, with a thiol- or amino-reactive label under mild conditions resulting in the labeling of said mutated protein phosphatase at the desired position in said structural element. Mild conditions refer to buffer pH (e.g. around pH=7 for thiol-reactive probes), ratio of label to protein phosphatase, temperature and length of the incubation step (for thiol-reactive probes e.g. 4 °C and overnight in the dark) which are known to the skilled person and provided with instruction manuals of providers of thiol- and amino-reactive probes. Such conditions may be fine-tuned in order to slow down the reaction of the chosen thiol- or amino-reactive label to ensure that labeling of said protein phosphatase is specific to the desired labeling site. In the case of fluorophore labeling, it is preferred to carry out the incubation in the dark. Increased light exposure may result in bleaching of the fluorophore and a less intense fluorescence emission. After labeling, the labeled protein phosphatase is preferably concentrated, purified by gel filtration, or washed several times with buffer to remove excess un-reacted label. The wash buffer is typically the buffer used to store the labeled protein phosphatase and may also be the buffer in which the desired measurements are made.

In a preferred embodiment of the method of generating a protein phosphatase according to the invention, said amino acid residues according to step (a), the labeling of which is not desirable, are solvent-exposed. In this preferred embodiment, only those reactive amino acids are changed which are likely to react with fluorophores, spin labels or isotope labels containing a reactive group, given their exposure to solvent.

In a preferred embodiment, the method of generating a protein phosphatase according to the invention further comprises (d) contacting the protein phosphatase obtained by the above method with an inhibitor of said protein phosphatase; and (e) recording the fluorescence emission signal, the EPR signal or the NMR signal of the label of the protein phosphatase; wherein a difference in the fluorescence, EPR or NMR signal is indicative of the protein phosphatase being suitable for the screening of protein phosphatase inhibitors.

While such difference is expected to occur for essentially all labeled protein phosphatases according to the invention, the latter embodiment is a means to determine those labeled protein phosphatases which are particularly suitable for any of the methods disclosed herein. For example, any inhibitor screen using such particularly sensitive labeled protein phosphatase may serve to screen a compound library in a particularly sensitive manner.

The figures show:

Figure 1: Wild-type sequences of preferred protein phosphatases. Structural elements according to the present invention, to the extent they are present, are highlighted.

Figure 2: Two preferred PTP1B mutants according to the present invention as compared to the wild-type sequence. Both in the WPD loop mutant (SEQ ID NO: 6) and the $\alpha 7$ helix mutant (SEQ ID NO: 7) cysteines at positions 32, 92, 121 and 215 have been replaced with serines in order to avoid inadvertent labeling at these positions in the course of preparing the labeled mutants.

Figure 3: Structural overview of PTP1b. The structure of PTP1b (wild type) is shown with critical structural features highlighted (A). Shown are the central helix $\alpha 4$, the C-terminal helix $\alpha 7$ adjacent to the allosteric site (site 2) and the WPD loop over the active site (site 1). The active site contains a conserved Cys residue near the end of $\alpha 4$ which is required for catalysis and dephosphorylation of substrates. An alignment of $\alpha 7$ and the allosteric sites of PTP1b and TC-PTP reveal highly conserved tertiary protein structure except for a critical difference in which only PTP1b has a Phe at a position which is favorable to ligand binding in this pocket. Both $\alpha 7$ helices of each protein contain a Trp which helps stabilize $\alpha 7$ against the phosphatase in the absence of allosteric inhibitors which act by displacing this Trp.

Figure 4: Scheme of the fluorescence-based binding assays for phosphatases. For the development of a binding assay which can detect conformational changes in certain phosphatase structural elements, we removed surface-exposed Cys residues to avoid non-specific acrylodan labeling. A Cys was inserted at the desired labeling position (F182C) in the WPD loop for an active site binding assay (scheme I) or at a position on helix $\alpha 7$ (L294C) for an assay to identify allosteric binders (scheme II). In Scheme I, the WPD loop will be stabilized in the down position upon ligand binding. In Scheme II, the helix will be displaced from the phosphatase upon ligand binding. Ligand binding to either site will induce a conformational change and alter the emission of acrylodan (sphere).

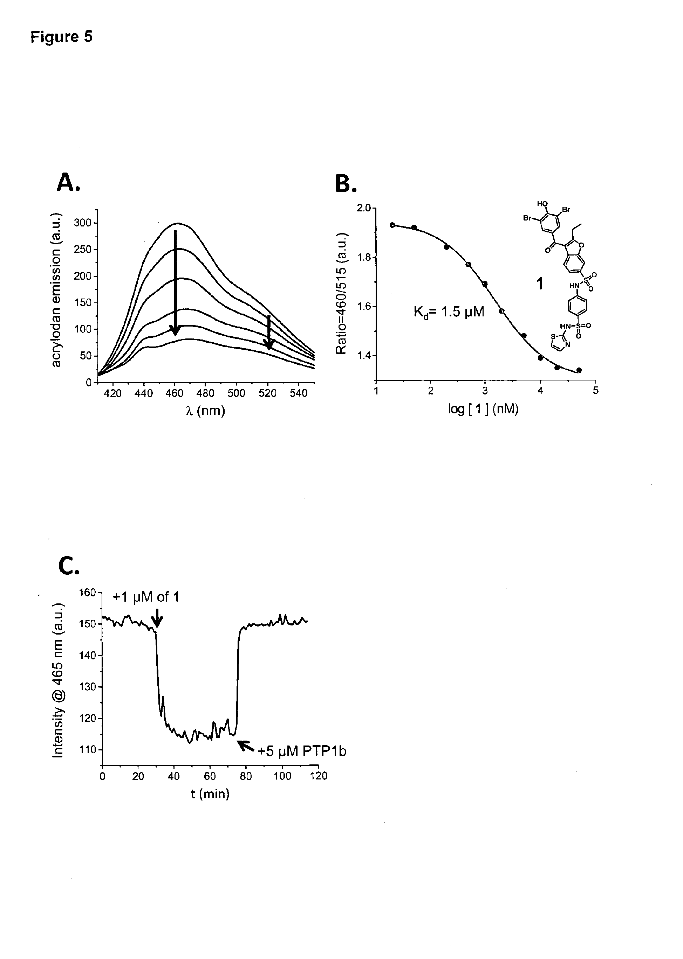

Figure 5: Hit compound **1** bound to the allosteric binding site of PTP1 b; see Example 1.

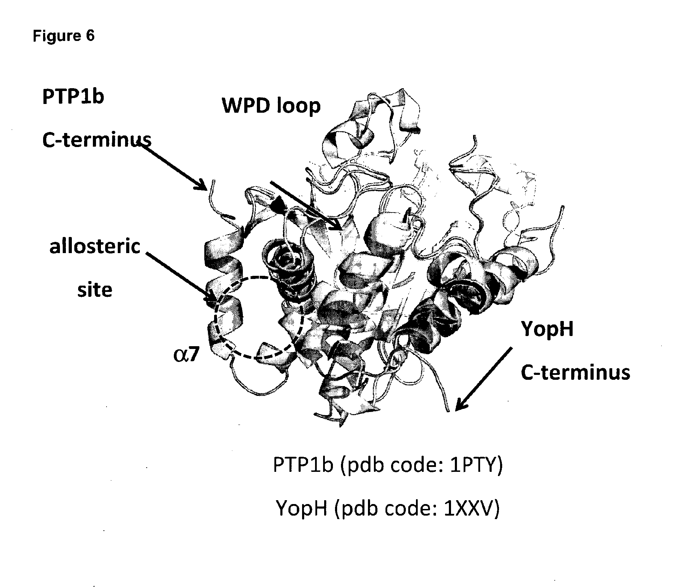

Figure 6: Structural comparison of PTP1b and YopH. The structure of PTP1b (wild type) is shown with critical structural features highlighted. Shown are the C-terminal helix $\alpha 7$ (teal) adjacent to the allosteric site (site 2) and the WPD loop (red) over the active site. An alignment of YopH (purple) with PTP1b (wheat) reveal several similarities in tertiary protein structure except for a critical difference in which only PTP1b has the C-terminal helix which interacts with an allosteric binding site. The C-terminus of YopH is truncated and this site does not exist, thus explaining why allosteric PTP1b inhibitors do not bind to YopH when used in the dissociation experiment shown in Figure 3C.

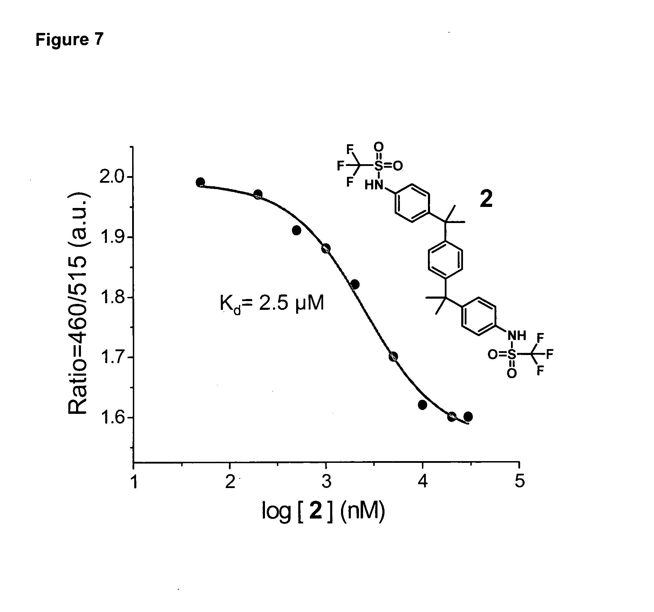

**Figure 7:** Hit compound **2** bound to the active site of PTP1 b; see Example 2.

The Examples illustrate the invention:

**Example 1**

Validation using an allosteric inhibitor of PTP1B

The compound (designated "**1**") was identified by monitoring acrylodan fluorescence changes upon ligand binding, revealing a robust loss in intensity at 460 nm relative to 515 nm (A). A ratiometric change in acrylodan emission at two wavelengths (R = 460 nm / 515 nm), allowing a mean $K_d$ of 1.25 $\mu$M to be determined (B). Using standard activity-based assays, this compound has a reported $IC_{50}$ of 8 $\mu$M. Addition of various non-phosphatase inhibitors (i.e. kinase inhibitors) were not detected by this labeling strategy, allowing specific detection of ligands which bind to phosphatases. The kinetics of the binding and dissociation of **1** were also examined by monitoring emission at a single wavelength (465 nm) over time (C). Addition of **1** caused a loss of fluorescence intensity while subsequent addition of a large excess of unlabeled PTP1b induced dissociation of **1** from the acrylodan-labeled PTP1b and restored fluorescence to a higher level. Interestingly, when the same dissociation experiment was attempted using YopH phosphatase to extract the bound ligand, a phosphatase which lacks the allosteric site found in PTP1 b, **1** did not dissociate and no fluorescence recovery was observed.

**Example 2**

Validation using an active site-binding inhibitor of PTP1B

The compound (designated "**2**") was identified by monitoring acrylodan fluorescence changes upon ligand binding as in Figure 3. A ratiometric change in acrylodan emission at two wavelengths (R = 460 nm / 515 nm) allowed a mean $K_d$ of 2.3 $\mu$M to be determined. Using standard activity-based assays, this compound has a reported $IC_{50}$ of 2.5 $\mu$M.

**Claims**

1. A protein phosphatase containing exactly one label in

   (a) a structural element of said protein phosphatase, said structural element being selected from

      (a)(i) the C-terminal helix; and
      (a)(ii) the WPD loop;

   (b) wherein said label is a fluorophore, a spin label or an isotope label,

      (b)(i) wherein said fluorophore and said spin label are covalently bound to a thiol group or side chain amino group of an amino acid residue of said structural element,
      (b)(ii) wherein said isotope label is a moiety comprising one or more atoms which are isotopes with non-zero nuclear spin, said moiety being covalently bound to a thiol group or side chain amino group of an amino acid residue of said structural element, or said isotope label is an amino acid residue of said structural element, said amino acid residue comprising one or more atoms which are isotopes with non-zero nuclear spin, wherein said isotopes with non-zero nuclear spin are enriched as compared to the natural occurrence thereof.

2. The protein phosphatase of claim 1, wherein said label in said structural element is the only fluorophore, spin label or isotope label present in said protein phosphatase.

3. The protein phosphatase of claim 1 or 2, wherein the amino acid sequence of said structural element carrying said label is

   (a) the amino acid sequence of the wild type form of said protein phosphatase;
   (b) differs from the amino acid sequence of said wild type form by the presence of one amino acid residue containing a thiol group or side chain amino group at a position where the amino acid sequence of the wild type does not contain such a residue; or
   (c) differs from the amino acid sequence of the wild type form by the absence of one or
   more amino acid residues containing a thiol group or side chain amino group,
   provided that exactly one amino acid residue or at least one amino acid residue containing a thiol group or side chain amino group is present in said structural element carrying said label.

4. The protein phosphatase of any one of claims 1 to 3, wherein

   (a) the amino acid sequence of said protein phosphatase outside said structural element is that of the wild type form; or
   (b) one, more or all amino acid residues containing a thiol group or side chain amino group and outside said structural element are deleted or replaced with amino acid residues which do not contain a thiol group or side chain amino group, wherein preferably said amino acid residues containing a thiol group or side chain amino group and outside said structural element are solvent-exposed.

5. The protein phosphatase according to claim 4(a) to the extent claim 4(a) refers back to claims 3(b), 2 and 1, wherein said protein phosphatase differs from the amino acid sequence of said wild type form by the presence of exactly one amino acid residue containing a thiol group or side chain amino group at a position where the amino acid sequence of the wild type does not contain such a residue.

6. The protein phosphatase according to claim 5, wherein said exactly one amino acid residue is a cysteine replacing an amino acid residue of said wild type form.

7. The protein phosphatase of any one of claims 1 to 6, wherein the amino acid sequence of said protein phosphatase or of the wild type form thereof is the amino acid sequence of PTP1 B, TCPTP, MptbB, SHP-1 or SHP-2.

8. The protein phosphatase of any one of claims 1 to 7, wherein the amino acid sequence of said protein phosphatase is the sequence set forth in SEQ ID NO: 6 or 7.

9. The protein phosphatase of claim 8, wherein there is exactly one label in said structural element, said label being acrylodan covalently bound to the thiol group of the cysteine residue at position 182 of SEQ ID NO: 6 or at position 294 or SEQ ID NO: 7.

10. A method of screening for ligands of the wild type form of the protein phosphatase defined in any one of claims 1 to 9, said method comprising:

    (a) contacting the protein phosphatase of any one of claims 1 to 9 with a candidate compound; and
    (b) recording the fluorescence emission signal, the EPR signal or the NMR signal of said
    label of said protein phosphatase of any one of claims 1 to 9,
    wherein a difference in signal as compared to the signal in absence of said candidate compound is indicative of said candidate compound being a ligand of the wild type form of the protein phosphatase defined in any one of claims 1 to 9.

11. A method of screening for inhibitors of the wild type form of the protein phosphatase defined in any one of claims 1 to 9, said method comprising the method of claim 10 and

    (c) determining the activity of said protein phosphatase defined in any one of claims 1 to 10 or of said wild type form thereof in the presence of said candidate compound,
    wherein a decrease in activity as compared to the absence of said candidate compound is indicative of said candidate compound being an inhibitor of the wild type form of the protein phosphatase defined in any one of claims 1 to 9.

12. A method of quantifying ligand or inhibitor binding to a protein phosphatase, said method comprising

 (a)

  (i) contacting a protein phosphatase according to any one of claims 1 to 9 with different concentrations of a ligand or inhibitor; and/or
  (ii) contacting a complex, the complex comprising or consisting of a protein phosphatase according to any one of claims 1 to 9 and a ligand or inhibitor, with different concentrations of unlabeled, but otherwise the same protein phosphatase or of the wild type form of said protein phosphatase;

 (b) recording the signal emitted by the label of said protein phosphatase according to any one of claims 1 to 9 at a given time point after said contacting and for each concentration of ligand or inhibitor according to (a)(i) and/or for each concentration of unlabeled or wild type form protein phosphatase according to (a)(ii); and
 (c) determining the dissociation and/or association constant of said complex from the concentration dependence of said signal recorded according to (b).

13. The method according to claim 12, wherein said label is a fluorophore and said signal is the ratio of emission intensities at two different wavelengths, the two wavelengths being local maxima of the emission spectrum.

14. A method of quantifying ligand or inhibitor binding to a protein phosphatase, said method comprising

 (a)

  (i) contacting a protein phosphatase according to any one of claims 1 to 9 with at least one concentration of a ligand or inhibitor; and/or
  (ii) contacting a complex, the complex comprising or consisting of a protein phosphatase according to any one of claims 1 to 9 and a ligand or inhibitor, with at least one concentration of unlabeled, but otherwise the same protein phosphatase or of the wild type form of said protein phosphatase;

 (b) recording the signal emitted by the label of said protein phosphatase according to any one of claims 1 to 9 as a function of time upon said contacting according to (a); and
 (c)

  (i) determining the rate constant governing the time dependence of said signal according to (b) for (each of) the concentration(s) according to (a)(i) and determining $k_{en}$ therefrom; and/or
  (ii) determining the rate constant governing the time dependence of said signal according to (b) for (each of) the concentration(s) according to (a)(ii) and determining $k_{off}$ therefrom.

15. The method of claim 14, further comprising calculating the dissociation constant and/or association constant from $k_{on}$ and $k_{off}$ as determined in step (c).

16. A method of generating a protein phosphatase suitable for the screening of protein phosphatase inhibitors, said method comprising

 (a) deleting one or more amino acid residues having a free thiol group or free side chain amino group, if any, present in the wild type form of said protein phosphatase, the labeling of said amino acid residues not being desirable, or replacing them with amino acid residues which do not contain a free thiol or side chain amino group, wherein preferably said amino acid residues the labeling of which is not desirable are solvent-exposed;
 (b) replacing an amino acid residue in a structural element as defined in claim 1 in said protein phosphatase with an amino acid residue containing a free thiol or side chain amino group if no amino acid residue containing a free thiol or side chain amino group is or would be present in said structural element after step (a), wherein steps (a) and (b) can be effected in any order; and
 (c) labeling the protein phosphatase obtained from steps (a) and (b) with a thiol- or amino-reactive fluorophore, thiol- or amino-reactive spin label, and/or thiol- or amino-reactive isotope label.

17. The method of claim 16, further comprising

(d) contacting the protein phosphatase obtained by the method of claim 16 with an inhibitor of said protein phosphatase; and

(e) recording the fluorescence emission signal, the EPR signal or the NMR signal of the label of the protein phosphatase;

wherein a difference in the fluorescence, EPR or NMR signal is indicative of the protein phosphatase being suitable for the screening of protein phosphatase inhibitors.

## Figure 1

Preferred Protein Phosphatases (wild-type sequences)

WPD-Loop and analogous highlighted underlined

Helix α7 highlighted in dashed boxes (only present in PTP1B and TCPTP)

**PTP1B (SEQ ID NO: 1)**

http://www.uniprot.org/uniprot/P18031

MEMEKEFEQIDKSGSWAAIYQDIRHEASDFPCRVAKLPKNKNRNRYRDVSPFDHSRIKLHQEDNDYINASLIKMEE

AQRSYILTQGPLPNTCGHFWEMVWEQKSRGVVMLNRVMEKGSLKCAQYWPQKEEKEMIFEDTNLKLTLISEDIKSY

YTVRQLELENLTTQETREILHFHYTTWPDFGVPESPASFLNFLFKVRESGSLSPEHGPVVVHCSAGIGRSGTFCLA

DTCLLLMDKRKDPSSVDIKKVLLEMRKFRMGLIQTADQLRFSYLAVIEGAKFIMGDSSVQDQWKELSHEDLEPPPE

HIPPPPRPPKRILEPHNGKCREFFPNHQWVKEETQEDKDCPIKEEKGSPLNAAPYGIESMSQDTEVRSRVVGGSLR

GAQAASPAKGEPSLPEKDEDHALSYWKPFLVNMCVATVLTAGAYLCYRFLFNSNT


**TCPTP (SEQ ID NO: 2)**

http://www.uniprot.org/uniprot/P17706

MPTTIEREFEELDTQRRWQPLYLEIRNESHDYPHRVAKFPENRNRNRYRDVSPYDHSRVKLQNAENDYINASLVDI

EEAQRSYILTQGPLPNTCCHFWLMVWQQKTKAVVMLNRIVEKESVKCAQYWPTDDQEMLFKETGFSVKLLSEDVKS

YYTVHLLQLENINSGETRTISHFHYTTWPDFGVPESPASFLNFLFKVRESGSLNPDHGPAVIHCSAGIGRSGTFSL

VDTCLVLMEKGDDINIKQVLLNMRKYRMGLIQTPDQLRFSYMAIIEGAKCIKGDSSIQKRWKELSKEDLSPAFDHS

PNKIMTEKYNGNRIGLEEEKLTGDRCTGLSSKMQDTMEENSESALRKRIREDRKATTAQKVQQMKQRLNENERKRK

RWLYWQPILTKMGFMSVILVGAFVGWTLFFQQNAL


**MptbB (SEQ ID NO: 3)**

http://www.uniprot.org/uniprot/P96830

MAVRELPGAWNFRDVADTATATALRPGRLFRSSELSRLDDAGRATLRRLGITDVADLRSSREVARRGPGRVPDGIDVH

LLPFPDLADDDADDSAPHETAFKRLLTNDGSNGESGESSQSINDAATRYMTDEYRQFPTRNGAQRALHRVVTLLAA

GRPVLTHCFAGKDRTGFVVALVLEAVGLDRDVIVADYLRSNDSVPQLRARISEMIQQRFDTELAPEVVTFTKARLS

DGVLGVRAEYLAAARQTIDETYGSLGGYLRDAGISQATVNRMRGVLLG


**SHP-1 (SEQ ID NO: 4)**

http://www.uniprot.org/uniprot/P29350

MVRWFHRDLSGLDAETLLKGRGVHGSFLARPSRKNQGDFSLSVRVGDQVTHIRIQNSGDFYDLYGGEKFATLTELV

EYYTQQQGVLQDRDGTIIHLKYPLNCSDPTSERWYHGHMSGGQAETLLQAKGEPWTFLVRESLSQPGDFVLSVLSD

QPKAGPGSPLRVTHIKVMCEGGRYTVGGLETFDSLTDLVEHFKKTGIEEASGAFVYLRQPYYATRVNAADIENRVL

ELNKKQESEDTAKAGFWEEFESLQKQEVKNLHQRLEGQRPENKGKNRYKNILPFDHSRVILQGRDSNIPGSDYINA

NYIKNQLLGPDENAKTYIASQGCLEATVNDFWQMAWQENSRVIVMTTREVEKGRNKCVPYWPEVGMQRAYGPYSVT

NCGEHDTTEYKLRTLQVSPLDNGDLIREIWHYQYLSWPDHGVPSEPGGVLSFLDQINQRQESLPHAGPIIVHCSAG

IGRTGTIIVIDMLMENISTKGLDCDIDIQKTIQMVRAQRSGMVQTEAQYKFIYVAIAQFIETTKKKLEVLQSQKGQ

ESEYGNITYPPAMKNAHAKASRTSSKHKEDVYENLHTKNKREEKVKKQRSADKEKSKGSLKRK

## Figure 1 continued

SHP-2 (SEQ ID NO: 5)

http://www.uniprot.org/uniprot/Q06124

MTSRRWFHPNITGVEAENLLLTRGVDGSFLARPSKSNPGDFTLSVRRNGAVTHIKIQNTGDYYDLYGGEKFATLA

ELVQYYMEHHGQLKEKNGDVIELKYPLNCADPTSERWFHGHLSGKEAEKLLTEKGKHGSFLVRESQSHPGDFVLS

VRTGDDKGESNDGKSKVTHVMIRCQELKYDVGGGERFDSLTDLVEHYKKNPMVETLGTVLQLKQPLNTTRINAAE

IESRVRELSKLAETTDKVKQGFWEEFETLQQQECKLLYSRKEGQRQENKNKNRYKNILPFDHTRVVLHDGDPNEP

VSDYINANIIMPEFETKCNNSKPKKSYIATQGCLQNTVNDFWRMVFQENSRVIVMTTKEVERGKSKCVKYWPDEY

ALKEYGVMRVRNVKESAAHDYTLRELKLSKVGQALLQGNTERTVWQYHFR**TWPD**HGV**P**SDPGGVLDFLEEVHHKQ

ESIMDAGPVVVHCSAGIGRTGTFIVIDILIDIIREKGVDCDIDVPKTIQMVRSQRSGMVQTEAQYRFIYMAVQHY

IETLQRRIEEEQKSKRKGHEYTNIKYSLADQTSGDQSPLPPCTPTPPCAEMREDSARVYENVGLMQQQKSFR


## Figure 2

### PTP1B wild-type and preferred mutants

WPD-Loop and analogous highlighted underlined

Helix α7 highlighted in dashed boxes (only present in PTP1B and TCPTP)

Mutations; single residues highlighted double underlined

### PTP1B wild type (truncated; SEQ ID NO: 8)

http://www.uniprot.org/uniprot/P18031

MEMEKEFEQIDKSGSWAAIYQDIRHEASDFPCRVAKLPKNKNRNRYRDVSPFDHSRIKLHQEDNDYINASLIKME

EAQRSYILTQGPLPNTCGHFWEMVWEQKSRGVVMLNRVMEKGSLKCAQYWPQKEEKEMIFEDTNLKLTLISEDIK

SYYTVRQLELENLTTQETREILHFHYT**TWPD**FGV**P**ESPASFLNFLFKVRESGSLSPEHGPVVVHCSAGIGRSGTF

CLADTCLLLMDKRKDPSSVDIKKVLLEMRKFRMGLIQTADQLRFSYLAVIEGAKFIMGDSSVQDQWKELSHEDLE

PPPEHIPPPPRPPKRILEPHN


### PTP1B WPD-Loop FLiP (C32S/C92S/C121S/C215S/F182C) (SEQ ID NO: 6)

MEMEKEFEQIDKSGSWAAIYQDIRHEASDFP**S**RVAKLPKNKNRNRYRDVSPFDHSRIKLHQEDNDYINASLIKME

EAQRSYILTQGPLPNT**S**GHFWEMVWEQKSRGVVMLNRVMEKGSLK**S**AQYWPQKEEKEMIFEDTNLKLTLISEDIK

SYYTVRQLELENLTTQETREILHFHYT**TWPDC**GV**P**ESPASFLNFLFKVRESGSLSPEHGPVVVH**S**SAGIGRSGTF

CLADTCLLLMDKRKDPSSVDIKKVLLEMRKFRMGLIQTADQLRFSYLAVIEGAKFIMGDSSVQDQWKELSHEDLE

PPPEHIPPPPRPPKRILEPHN


### PTP1B helix α7 FLiP (C32S/C92S/C121S/C215S/L294C) (SEQ ID NO: 7)

MEMEKEFEQIDKSGSWAAIYQDIRHEASDFP**S**RVAKLPKNKNRNRYRDVSPFDHSRIKLHQEDNDYINASLIKME

EAQRSYILTQGPLPNT**S**GHFWEMVWEQKSRGVVMLNRVMEKGSLK**S**AQYWPQKEEKEMIFEDTNLKLTLISEDIK

SYYTVRQLELENLTTQETREILHFHYT**TWPD**FGV**P**ESPASFLNFLFKVRESGSLSPEHGPVVVH**S**SAGIGRSGTF

CLADTCLLLMDKRKDPSSVDIKKVLLEMRKFRMGLIQTADQLRFSYLAVIEGAKFIMGDSSVQDQWKE**C**SHEDLE

PPPEHIPPPPRPPKRILEPHN

**Figure 3**

A.

WPD loop

Catalytic Cys

α7

2

α4

Human PTP1b (pdb code: 1PTY)

B.

TC-PTP     PTP1b

**Figure 4**

I.

II.

**Figure 5**

Figure 6

PTP1b (pdb code: 1PTY)

YopH (pdb code: 1XXV)

**Figure 7**

**EP 2 565 276 A1**

Europäisches
Patentamt

European
Patent Office

Office européen
des brevets

## EUROPEAN SEARCH REPORT

Application Number

EP 11 00 7082

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHANG X Y ET AL: "Allele-specific inhibition of divergent protein tyrosine phosphatases with a single small molecule", BIOORGANIC & MEDICINAL CHEMISTRY, PERGAMON, GB, vol. 16, no. 17, 1 September 2008 (2008-09-01), pages 8090-8097, XP024524928, ISSN: 0968-0896, DOI: 10.1016/J.BMC.2008.07.053 [retrieved on 2008-07-24] | 1-9 | INV.<br>C12Q1/42 |
| A | * abstract; fig.1 * | 10-17 | |
| X | WO 03/087051 A2 (SUNESIS PHARMACEUTICALS INC [US]; ERLANSON DANIEL A [US]; HANSEN STIG) 23 October 2003 (2003-10-23) | 1-9,16, 17 | |
| A | * p.12, line 33-p.15, line 5; p.23, line 20-p.25, line 17 * | 10-15 | |
| A | WIESMANN CHRISTIAN ET AL: "Allosteric inhibition of protein tyrosine phosphatase 1B", NATURE STRUCTURAL & MOLECULAR BIOLOGY, vol. 11, no. 8, August 2004 (2004-08), pages 730-737, XP002672022, ISSN: 1545-9993 * the whole document * | 1-17 | TECHNICAL FIELDS SEARCHED (IPC)<br><br>C12Q |
| A | MACHLEIDT THOMAS ET AL: "Protein labeling with FlAsH and ReAsH.", METHODS IN MOLECULAR BIOLOGY (CLIFTON, N.J.) 2007 LNKD- PUBMED:16988405, vol. 356, 2007, pages 209-220, XP009157719, ISSN: 1064-3745 * fig.1 * | 1-17 | |

-/--

The present search report has been drawn up for all claims

| Place of search | Date of completion of the search | Examiner |
|---|---|---|
| Munich | 23 March 2012 | Pellegrini, Paolo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 2 565 276 A1

Europäisches
Patentamt

European
Patent Office

Office européen
des brevets

## EUROPEAN SEARCH REPORT

Application Number

EP 11 00 7082

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2005/114197 A2 (EINSTEIN COLL MED [US]; ZHANG ZHONG-YIN [US]; KUMAR SANJAI [US]) 1 December 2005 (2005-12-01) * abstract; p.8, lines 8-21; p.22, lines 19-31; claims 36-38 * <br> ----- | 1-17 | |

TECHNICAL FIELDS SEARCHED (IPC)

The present search report has been drawn up for all claims

| Place of search | Date of completion of the search | Examiner |
|---|---|---|
| Munich | 23 March 2012 | Pellegrini, Paolo |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 00 7082

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2012

| Patent document cited in search report | | Publication date | Patent family member(s) | | Publication date |
|---|---|---|---|---|---|
| WO 03087051 | A2 | 23-10-2003 | AU | 2003224893 A1 | 27-10-2003 |
| | | | CA | 2478395 A1 | 23-10-2003 |
| | | | EP | 1497648 A2 | 19-01-2005 |
| | | | JP | 2006503264 A | 26-01-2006 |
| | | | WO | 03087051 A2 | 23-10-2003 |
| WO 2005114197 | A2 | 01-12-2005 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ANDERSEN et al.** *Molecular and Cellular Biology,* 2001, vol. 21, 7117-7136
- **BARR et al.** *Cell,* 2009, vol. 136, 352-363
- **KAMERLIN et al.** *BBRC,* 2007, vol. 356, 1011-1016
- **WIESMANN et al.** *Nature, structural & molecular biology,* 2004, vol. 11, 730-737
- **VINTONYAK et al.** *Tetrahedron,* 2011, vol. 67, 6713-6729